(19) Europäisches Patentamt
European Patent Office
Office européen des brevets

(11) EP 4 535 484 A1

(12) **EUROPEAN PATENT APPLICATION**
published in accordance with Art. 153(4) EPC

(43) Date of publication:
**09.04.2025 Bulletin 2025/15**

(21) Application number: **22943102.8**

(22) Date of filing: **25.05.2022**

(51) International Patent Classification (IPC):
***H01M 10/0525** (2010.01)* ***H01M 10/056** (2010.01)*
***H01M 10/0567** (2010.01)*

(52) Cooperative Patent Classification (CPC):
**H01M 10/0567; C07C 69/14; C07C 69/24; C07C 69/96; H01M 10/052; H01M 10/0525; H01M 10/056; H01M 10/0568; H01M 10/0569; H01M 10/0587;** H01M 2300/0034; Y02E 60/10

(86) International application number:
**PCT/CN2022/094915**

(87) International publication number:
**WO 2023/225897 (30.11.2023 Gazette 2023/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ningde Amperex Technology Limited**
**Ningde, Fujian 352100 (CN)**

(72) Inventors:
- **WANG, Xiang**
  **Ningde, Fujian 352100 (CN)**
- **WANG, Rui**
  **Ningde, Fujian 352100 (CN)**
- **TANG, Chao**
  **Ningde, Fujian 352100 (CN)**

(74) Representative: **Klunker IP Patentanwälte PartG mbB**
**Destouchesstraße 68**
**80796 München (DE)**

(54) **ELECTROCHEMICAL DEVICE AND ELECTRONIC DEVICE COMPRISING SAME**

(57) This application relates to an electrochemical apparatus and an electronic apparatus including the same. The electrochemical apparatus includes an electrolyte, where the electrolyte includes a carboxylate compound and fluoroethylene carbonate (FEC). Based on a total weight of the electrolyte, percentages of the carboxylate compound and FEC are $w_1$ and $w_2$ respectively, where $5\% \leq w_1 \leq 60\%$, $2\% \leq w_2 \leq 12\%$, and $2 \leq w_1/w_2 \leq 20$. The electrochemical apparatus delivers excellent fast charging performance and high-temperature interval cycle performance.

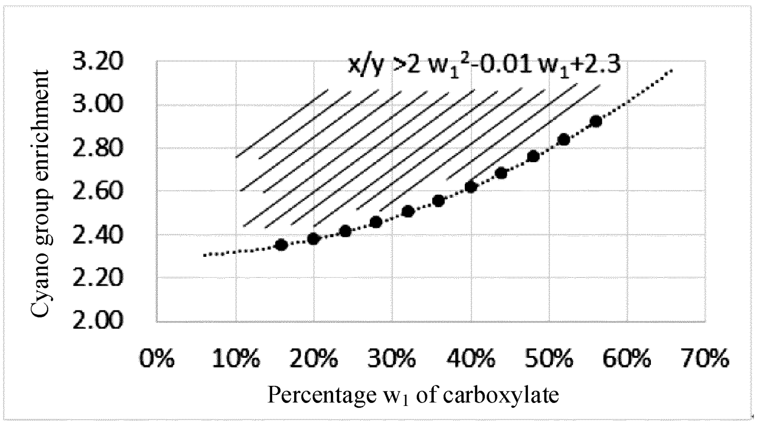


FIG. 1



EP 4 535 484 A1

## Description

### TECHNICAL FIELD

This application relates to the field of energy storage technologies, and more specifically, to an electrochemical apparatus and an electronic apparatus containing the same.

### BACKGROUND

With advantages such as high energy density, relatively simple reaction mechanism, high operating voltage, long service life, and environmental friendliness, rechargeable electrochemical apparatuses are considered to be one of the most attractive energy storage systems. Currently, electrochemical apparatuses such as lithium-ion batteries have been widely used in electronic products such as notebook computers, smart phones, and wearable devices.

With pursuit of fast charging of electronic products in the market, increasingly high requirements are imposed on charging speeds of electrochemical apparatuses, in which case the electrochemical apparatuses are required to still have excellent electrochemical performance, especially excellent cycling performance, at a high-rate current density. In addition, fast charging of electrochemical apparatuses is usually accompanied by heat generation, and temperature rise further deteriorates the cycling stability of the electrochemical apparatuses. For example, notebook computers in use are generally charged to a full charge state, then kept at the full charge state for several hours, and finally disconnected from chargers to enter a discharge state. Such process causes batteries in the notebook computers to heat up and thus be in a high-temperature state. When the notebook computers undergo intermittent operating conditions of high-temperature storage and charge and discharge cycles, the battery capacity is seriously degraded, which in turn imposes higher requirements on the high-temperature interval cycle (Interval Cycle, ITC) performance of electrochemical apparatuses.

In view of this, it is urgent to obtain an electrochemical apparatus with excellent fast charging performance and high-temperature interval cycle performance to meet such requirements of people.

### SUMMARY

To at least resolve the foregoing problem, this application improves formulation composition of an electrolyte and/or designs a size of an electrochemical apparatus to improve fast charging performance and high-temperature interval cycle performance of the electrochemical apparatus.

According to an aspect of this application, this application provides an electrolyte. The electrolyte includes a carboxylate compound represented by formula (I) and fluoroethylene carbonate (FEC),

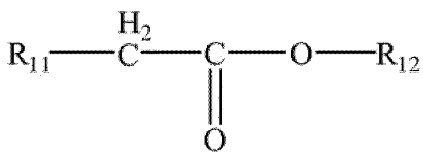

formula (I),

where $R_{11}$ includes at least one of hydrogen, a hydroxyl group, a C1-C20 alkyl group, a C1-C20 alkoxy group, a C2-C20 linear alkenyl group, a C6-C30 aryl group, or a C6-C30 aryloxy group, and $R_{12}$ includes at least one of a C1-C20 alkyl group, a C2-C20 linear alkenyl group, or a C6-C30 aryl group; and

based on a total weight of the electrolyte, percentages of the carboxylate compound represented by formula (I) and FEC are $w_1$ and $w_2$ respectively, where $5\% \leq w_1 \leq 60\%$, $2\% \leq w_2 \leq 12\%$, and $2 \leq w_1/w_2 \leq 20$.

According to an embodiment of this application, $4 \leq w_1/w_2 \leq 10$.

According to an embodiment of this application, the carboxylate compound represented by formula (I) includes at least one of the following: methyl acetate, ethyl acetate, propyl acetate, methyl propionate, ethyl propionate, propyl propionate, isopropyl propionate, butyl propionate, isobutyl propionate, n-pentyl propionate, isoamyl propionate, ethyl butyrate, propyl butyrate, propyl isobutyrate, amyl butyrate, amyl isobutyrate, butyl butyrate, isobutyl isobutyrate, or pentyl valerate.

According to an embodiment of this application, the carboxylate compound represented by formula (I) includes propyl propionate and ethyl acetate.

According to an embodiment of this application, the electrolyte further includes a nitrile compound; and based on a total weight of the electrolyte, a percentage of the nitrile compound is $w_3$, where $0.1\% \leq w_3 \leq 12\%$.

According to an embodiment of this application, the nitrile compound includes at least one of compounds represented by formula (II) to formula (V):

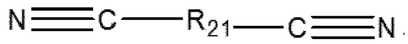

formula (II),

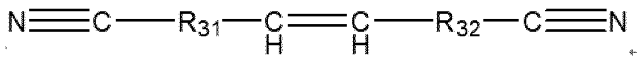

formula (III),

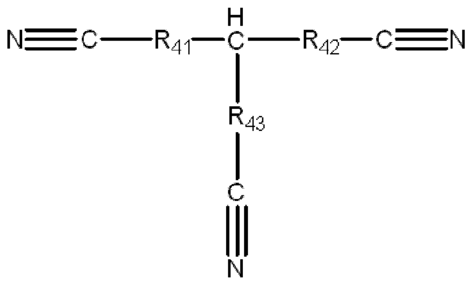

formula (IV), or

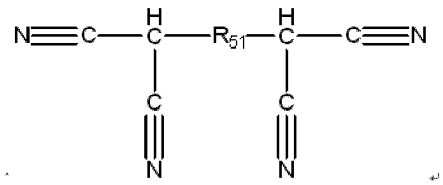

formula (V),

where $R_{21}$ includes at least one of a substituted or unsubstituted C1-C12 alkylidene group or a substituted or unsubstituted C1-C12 alkyleneoxy group;

$R_{31}$ and $R_{32}$ each independently include hydrogen and a substituted or unsubstituted C1-C12 alkylidene group;

$R_{41}$, $R_{42}$, and $R_{43}$ each independently include hydrogen, a substituted or unsubstituted C1-C12 alkylidene group, or a substituted or unsubstituted C1-C12 alkyleneoxy group; and

$R_{51}$ includes a substituted or unsubstituted C1-C12 alkylidene group, a substituted or unsubstituted C2-C12 alkenylene group, a substituted or unsubstituted C6-C26 arylidene group, or a substituted or unsubstituted C2-C12 heterocyclylene group, where a heteroatom is at least one of N, S, or O; where

during substitution, a substituent group is halogen.

According to an embodiment of this application, a total molar mass of cyano groups (-CN) in the nitrile compound is x, a total molar mass of the nitrile compound is y, and cyano group enrichment x/y satisfies $2.16 \leq x/y \leq 2.71$.

According to an embodiment of this application, the cyano group enrichment x/y, the percentage $w_1$ of the carboxylate compound represented by formula (I), and the percentage $w_2$ of FEC satisfy $2w_1^2 - 0.01w_1 + 2.3 > x/y > 27w_2^2 - 1.2w_2 + 2.1$.

According to an embodiment of this application, the electrolyte includes a lithium salt, and the lithium salt includes at least one of the following: $LiPF_6$, $LiBF_4$, $LiAsF_6$, $LiClO_4$, $LiB(C_6H_5)_4$, $LiCH_3SO_3$, $LiCF_3SO_3$, $LiN(SO_2CF_3)_2$, $LiC(SO_2CF_3)_3$, $LiSiF_6$, LiBOB, or LiDFOB.

According to another aspect of this application, this application further provides an electrochemical apparatus, including the electrolyte according to the foregoing embodiments.

According to an embodiment of this application, the electrochemical apparatus further includes a positive electrode, a negative electrode, and a separator sandwiched between the positive electrode and the negative electrode, where the positive electrode, the negative electrode, and the separator are wound to form a cell, and a length L and a width

W of the cell satisfy 20 mm $\leq L \leq$ 300 mm, 20 mm $\leq W \leq$ 100 mm, and $1 \leq L/W \leq 4$.

According to an embodiment of this application, the electrochemical apparatus satisfies at least one of the following characteristics: (a) $1 \leq L/W \leq 3$; (b) $2 \leq L/W \leq 3$; and (c) $2 \leq L/W \leq 4$.

According to an embodiment of this application, the electrochemical apparatus further includes a positive electrode, a negative electrode, and a separator sandwiched between the positive electrode and the negative electrode, where the positive electrode, the negative electrode, and the separator are wound to form a cell, and a thickness T and a width W of the cell satisfy 2 mm $\leq T \leq$ 12 mm, and $W/T \geq 5$.

According to an embodiment of this application, the electrochemical apparatus satisfies at least one of the following characteristics: (d) $5 \leq W/T \leq 25$; (e) $5 \leq W/T \leq 20$; (f) $5 \leq W/T \leq 15$; and (g) $10 \leq W/T \leq 25$.

According to an embodiment of this application, the electrochemical apparatus further includes a positive electrode, a negative electrode, and a separator sandwiched between the positive electrode and the negative electrode, where the positive electrode, the negative electrode, and the separator are wound to form a cell, and a length L and a width W of the cell and the percentage $w_1$ of the carboxylate compound represented by formula (I) satisfy $w_1 \times 100/(L/W) \geq 10$.

According to an embodiment of this application, the electrochemical apparatus satisfies at least one of the following characteristics: (h) $10 \leq w_1 \times 100/(L/W) \leq 40$; (i) $20 \leq w_1 \times 100/(L/W) \leq 30$; (j) $20 \leq w_1 \times 100/(L/W) \leq 40$; and (k) $15 \leq w_1 \times 100/(L/W) \leq 30$.

According to another aspect of this application, this application further provides an electronic apparatus, including the electrochemical apparatus according to the foregoing embodiments.

## BRIEF DESCRIPTION OF DRAWINGS

To describe some embodiments of this application, the following briefly describes the accompanying drawings required for describing some embodiments of this application or the prior art. Apparently, the accompanying drawings described below are merely some embodiments of this application. Persons skilled in the art can still derive drawings of other embodiments from structures shown in these accompanying drawings without creative efforts.

FIG 1 is a relationship diagram of cyano group enrichment x/y and a percentage $w_1$ of a carboxylate compound.
FIG 2 is a relationship diagram of cyano group enrichment x/y and a percentage $w_2$ of fluoroethylene carbonate (FEC).
FIG 3 shows length L, width W, and thickness T of a packaged cell.

## DESCRIPTION OF EMBODIMENTS

Some embodiments of this application are described in detail below. Some embodiments of this application should not be construed as any limitation on this application.

As in this application, the terms "include", "contain", and "comprise" are used in their inclusive and non-limiting meanings.

In addition, quantities, ratios, and other values are sometimes presented in the format of range in this specification. It should be understood that such format of range is used for convenience and simplicity and should be flexibly understood as including not only values explicitly designated as falling within the range but also all individual values or sub-ranges covered by the range as if each value and sub-range are explicitly designated.

In specific embodiments and claims, a list of items connected by the terms "one or more of", "one or more pieces of", "at least one of" or other similar terms may mean any combination of the listed items. For example, if items A and B are listed, the phrase "at least one of A or B" means only A; only B; or A and B. In another example, if items A, B, and C are listed, the phrase "at least one of A, B, or C" means only A; only B; only C; A and B (exclusive of C); A and C (exclusive of B); B and C (exclusive of A); or all of A, B, and C. The item A may include a single element or a plurality of elements. The item B may include a single element or a plurality of elements. The item C may include a single element or a plurality of elements.

The term "alkyl group" covers linear and branched alkyl groups. For example, the alkyl group may be a C1-C50 alkyl group, a C1-C40 alkyl group, a C1-C30 alkyl group, a C1-C20 alkyl group, a C1-C12 alkyl group, a C1-C10 alkyl group, a C1-C6 alkyl group, a C2-C6 alkyl group, or a C2-C5 alkyl group. In some embodiments, the alkyl group includes a methyl group, an ethyl group, a propyl group, an isopropyl group, a butyl group, an isobutyl group, a tert-butyl group, a pentyl group, a hexyl group, a heptyl group, an octyl group, and the like. In addition, the alkyl group may be arbitrarily substituted.

The term "alkenyl group" covers linear and branched alkenyl groups. For example, the alkenyl group may be a C2-C50 alkenyl group, a C2-C40 alkenyl group, a C2-C30 alkenyl group, a C2-C20 alkenyl group, a C2-C12 alkenyl group, a C2-C10 alkenyl group, or a C2-C6 alkenyl group. In addition, the alkenyl group may be arbitrarily substituted.

The term "aryl group" covers monocyclic and polycyclic systems. A polycyclic ring may have two or more rings in which two carbons are shared by two adjacent rings (the rings are "fused"), where at least one of the rings is aromatic, and other rings may be, for example, a cycloalkyl group, a cycloalkenyl group, an aryl group, a heterocyclic and/or heteroaryl group. For example, the aryl group may be a C6-C50 aryl group, a C6-C40 aryl group, a C6-C30 aryl group, a C6-C20 aryl

group, or a C6-C10 aryl group. In addition, the aryl group may be arbitrarily substituted.

The term "alkoxy group" is an organic group having -O-R1, where R1 is the above-mentioned linear or branched alkyl group.

The term "aryloxy group" is an organic group having -O-R2, where R2 is the above-mentioned aryl group.

The term "alkylidene group" covers linear and branched alkylidene groups. For example, the alkylidene group may be a C1-C50 alkylidene group, a C1-C40 alkylidene group, a C1-C30 alkylidene group, a C1-C20 alkylidene group, a C1-C10 alkylidene group, a C1-C6 alkylidene group, a C2-C6 alkylidene group, or a C2-C5 alkylidene group. In addition, the alkylidene group may be arbitrarily substituted.

The term "alkenylene group" covers linear and branched alkenylene groups. For example, the alkenylene group may be a C2-C50 alkenylene group, a C2-C40 alkenylene group, a C2-C30 alkenylene group, a C2-C20 alkenylene group, a C2-C10 alkenylene group, a C1-C6 alkenylene group, or a C2-C6 alkenylene group. In addition, the alkenylene group may be arbitrarily substituted.

The term "heterocyclylene group" includes a closed-ring structure similar to a carbocyclic group in which one or more of carbon atoms on a ring are non-carbon elements such as nitrogen, sulfur, or oxygen. The heterocyclylene group includes but is not limited to an aziridine group, an epoxyethane group (epoxide or epoxyethane), an ethylene sulfone group (episulfide), a bisepoxyethane group, an azetidine group, an oxetane group, a thiacyclobutane group, a dioxetane group, a dithietane group, a dithiocyclobutene group, an azacyclopentane group, a pyrrolidine group, a pyrroline group, a tetrahydrofuran group, a dihydrofuran group, and a furan group.

When the foregoing groups are substituted, substituent groups may be each independently selected from an alkyl group, an alkenyl group, an aryl group, an alkoxy group, an aryloxy group, a silane group, a siloxy group, an amino group, an ether group, an ester group, a carboxyl group, a sulfonic acid group, a sulfydryl group, a cyano group, halogen, or a combination thereof.

## I. Electrolyte

As an important part of an electrochemical apparatus, an electrolyte is used to conduct lithium ions between positive and negative electrodes so as to implement continuous intercalation and deintercalation of the lithium ions in positive and negative electrode materials, thereby implementing charging and discharging functions. Therefore, the electrolyte has a crucial effect on the electrochemical performance of the electrochemical apparatus.

To improve fast charging performance of the electrochemical apparatus, a carboxylate compound with a low viscosity is added into the electrolyte in this application, so as to improve the transmission rate of the lithium ions in the electrolyte. However, it is further found in this application that the carboxylate compound has a narrow electrochemical window and poor high-temperature stability. For example, when an electronic apparatus (for example, a notebook computer) undergoes intermittent operating conditions of high-temperature storage and charge and discharge cycles, the carboxylate compound reacts at a negative electrode to consume active lithium and cause damage to a solid electrolyte interface (SEI) film on the surface of the negative electrode, thus leading to rapid degradation of battery capacity.

To effectively alleviate the damage of the carboxylate compound to the SEI film on the negative electrode and repair the SEI film on the negative electrode in a timely manner, fluoroethylene carbonate (FEC) is further added into the electrolyte in this application. However, it is further found in this application that when an electrochemical apparatus undergoes intermittent operating conditions of high-temperature storage and charge and discharge cycles, a positive electrode structure is easily damaged and oxygen release reactions take place, in which case FEC is easily oxidized and decomposed to generate $CO_2$, leading to swelling and gas generation of the electrochemical apparatus.

However, it is unexpectedly found in this application that adjusting percentages of the carboxylate compound and FEC in the electrolyte and a ratio thereof can effectively improve the fast charging performance of the electrochemical apparatus, without deteriorating or slightly deteriorating the high-temperature interval cycle performance of the electrochemical apparatus. In some embodiments, based on a total weight of the electrolyte, percentages of the carboxylate compound and FEC are $w_1$ and $w_2$ respectively, where $5\% \leq w_1 \leq 60\%$, $2\% \leq w_2 \leq 12\%$, and $2 \leq w_1/w_2 \leq 20$.

In some embodiments, $w_1$ may be but is not limited to 5%, 10%, 15%, 20%, 25%, 30%, 35%, 40%, 45%, 50%, 55%, or 60%, or falls within a range defined by any two of the foregoing values. For example, $10\% \leq w_1 \leq 50\%$ or $15\% \leq w_1 \leq 50\%$.

In some embodiments, $w_2$ may be but is not limited to 2%, 4%, 6%, 8%, 10%, or 12%, or falls within a range defined by any two of the foregoing values. For example, $4\% \leq w_2 \leq 12\%$ or $4\% \leq w_1 \leq 10\%$.

In some embodiments, $w_1/w_2$ may be but is not limited to 2, 4, 6, 8, 10, 12, 14, 16, 18, or 20, or falls within a range defined by any two of the foregoing values. For example, $4 \leq w_1/w_2 \leq 16$ or $4 \leq w_1/w_2 \leq 10$.

In some embodiments, the carboxylate compound is a carboxylate compound represented by the following formula (I):

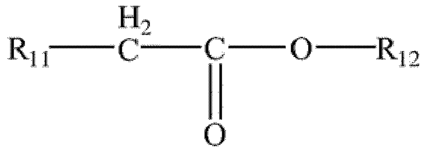

formula (I),

where $R_{11}$ includes at least one of hydrogen, a hydroxyl group, a C1-C20 alkyl group, a C1-C20 alkoxy group, a C2-C20 linear alkenyl group, a C6-C30 aryl group, or a C6-C30 aryloxy group, and $R_{12}$ includes at least one of a C1-C20 alkyl group, a C2-C20 linear alkenyl group, or a C6-C30 aryl group.

In some embodiments, the carboxylate compound represented by formula (I) includes at least one of the following: methyl acetate, ethyl acetate, propyl acetate, methyl propionate, ethyl propionate, propyl propionate, isopropyl propionate, butyl propionate, isobutyl propionate, n-pentyl propionate, isoamyl propionate, ethyl butyrate, propyl butyrate, propyl isobutyrate, amyl butyrate, amyl isobutyrate, butyl butyrate, isobutyl isobutyrate, or pentyl valerate.

In some embodiments, the carboxylate compound represented by formula (I) includes at least propyl propionate. Compared with other carboxylate compounds, propyl propionate is more conducive to stabilizing a positive electrode structure, and therefore can improve the high-temperature ITC performance of the electrochemical apparatus and suppress gas generation while improving the fast charging performance of the electrochemical apparatus, thereby enhancing overall performance of the electrochemical apparatus.

In some embodiments, the carboxylate compound represented by formula (I) includes ethyl propionate and ethyl acetate. In some embodiments, the carboxylate compound represented by formula (I) includes propyl propionate and ethyl acetate. In some embodiments, the carboxylate compound represented by formula (I) includes propyl propionate and ethyl propionate. In some embodiments, the carboxylate compound represented by formula (I) includes ethyl acetate, ethyl propionate, and propyl propionate.

In some embodiments, the electrolyte further includes a nitrile compound. The nitrile compound includes cyano (-CN) functional groups. In some embodiments, based on the total weight of the electrolyte, a percentage of the nitrile compound is $w_3$, where $0.1\% \leq w_3 \leq 12\%$. In some embodiments, $w_3$ may be but is not limited to 0.1%, 1%, 2%, 4%, 6%, 8%, 10%, or 12%, or falls within a range defined by any two of the foregoing values. When the percentage of the nitrile compound falls within the foregoing range, the high-temperature ITC performance of the electrochemical apparatus can be improved and gas generation can be suppressed while the fast charging performance of the electrochemical apparatus is guaranteed. This is because the nitrile compound can form a nitrile protective film with excellent performance on the surface of a positive electrode, thereby well stabilizing active metals in a positive electrode active material, inhibiting dissolution of the active metals, and reducing oxygen release reactions.

In some embodiments, the nitrile compound includes at least one of compounds represented by formula (II) to formula (V):

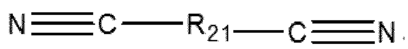

formula (II),

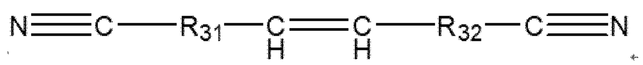

formula (III),

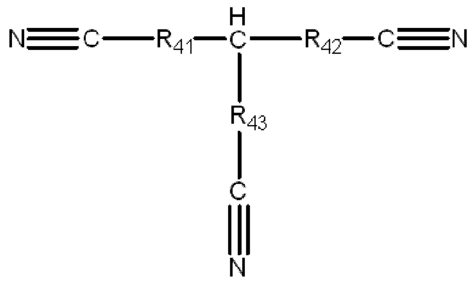

formula (IV), or

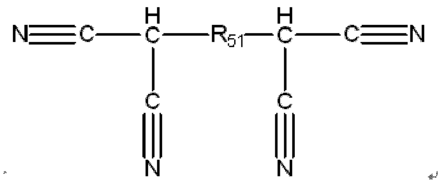

formula (V),

where $R_{21}$ includes at least one of a substituted or unsubstituted C1-C12 alkylidene group or a substituted or unsubstituted C1-C12 alkyleneoxy group;

$R_{31}$ and $R_{32}$ each independently include hydrogen and a substituted or unsubstituted C1-C12 alkylidene group;

$R_{41}$, $R_{42}$, and $R_{43}$ each independently include hydrogen, a substituted or unsubstituted C1-C12 alkylidene group, or a substituted or unsubstituted C1-C12 alkyleneoxy group; and

$R_{51}$ includes a substituted or unsubstituted C1-C12 alkylidene group, a substituted or unsubstituted C2-C12 alkenylene group, a substituted or unsubstituted C6-C26 arylidene group, or a substituted or unsubstituted C2-C12 heterocyclylene group, where a heteroatom is at least one of N, S, or O; where

during substitution, a substituent group is halogen.

In some embodiments, the nitrile compound includes at least one of hexanedinitrile, butanedinitrile, pentanedinitrile, malonononitrile, 2-methylglutaronitrile, heptanedinitrile, decanedinitrile, nonanedinitrile, 1,4-dicyano-2-butene, ethylene glycol bis(propanenitrile) ether, 3,3'-oxydipropionitrile, thiomalononitrile, hex-2-enedinitrile, butenedinitrile, 2-pentenedinitrile, ethylbutanedinitrile, hex-3-enedinitrile, 2-methyleneglutaronitrile, 4-cyanopilmonitrile, 1,3,6-hexanetricarbonitrile, 1,3,5-trimethylhexahydronitrile, 1,2,3-propanetricarbonitrile, or 1,2,3-tris(2-cyanoxyl)propane.

In this application, the effect of cyano group enrichment on electrochemical performance is also evaluated, where the cyano group enrichment is a ratio of a total molar mass x of cyano groups (-CN) in the nitrile compound to a total molar mass y of the nitrile compound. It is found in this application that as compared with a single nitrile compound, for example, a dinitrile compound (that is, $x/y = 2$) or a trinitrile compound (that is, $x/y = 3$), being added into the electrolyte, when the cyano group enrichment x/y satisfies $2.16 \leq x/y \leq 2.71$, the high-temperature ITC performance of the electrochemical apparatus can be significantly improved and gas generation can be suppressed. In addition, the deterioration of the fast charging performance of the electrochemical apparatus caused by the nitrile compound can also be greatly reduced. Therefore, the fast charging performance and the high-temperature ITC performance are expected to be simultaneously improved. This may be because as compared with thick SEI films formed by macromolecular nitriles with a large number of cyano groups (for example, trinitrile compounds) and less stable SEI films formed by small-molecule nitriles with a small number of cyano groups (for example, dinitrile compounds), a variety of nitriles with different numbers of cyano groups being added into the electrolyte can form macromolecular and small-molecule composite nitrile-containing SEI films on the surface of the positive electrode, achieving a better effect.

In addition, in this application, relationships between the percentage $w_1$ of the carboxylate compound, the percentage $w_2$ of FEC, and the cyano group enrichment x/y are also researched. Specifically, a curve represented by circular points in FIG 1 is $x/y = 2w_1^2 - 0.01w_1 + 2.3$, and a curve represented by square points in FIG 2 is $x/y = 27w_2^2 - 1.2w_2 + 2.1$. It is found through researches in this application that when the cyano group enrichment satisfies $x/y > 2w_1^2 - 0.01w_1 + 2.3$, the concentration of the cyano groups is high, which increases viscosity of the electrolyte to some extent and hinders transmission of lithium ions as well as intercalation and deintercalation of the lithium ions in positive and negative electrode active substances, thus increasing the probability of the lithium ions precipitating at positive and negative electrodes to form lithium dendrites. When the cyano group enrichment satisfies $x/y < 27w_2^2 - 1.2w_2 + 2.1$, the concentration of the cyano

groups is low, an SEI film formed on an active surface of the positive electrode has a slightly poor structural stability, and thus the probability of swelling and gas generation is increased.

Therefore, when the relationship among the percentage $w_1$ of the carboxylate compound, the percentage $w_2$ of FEC, and the cyano group enrichment x/y satisfies $2w_1^2-0.01w_1 + 2.3 > x/y > 27w_2^2 - 1.2w_2 + 2.1$, the viscosity of the electrolyte can be kept within a more appropriate range, transmission of the lithium ions in the electrolyte can be improved, and the risk of lithium precipitation can be significantly reduced, thereby improving the fast charging performance of the electrochemical apparatus. In addition, complexation protection of the nitrile compound for transition metals in the positive electrode active substance can be further enhanced, oxygen released at the positive electrode under the high-temperature interval cycle condition can be reduced, and the structural stability of the positive electrode can be improved, thereby improving the high-temperature ITC performance of the electrochemical apparatus and suppressing gas generation. That is, when the electrochemical apparatus satisfies $2w_1^2 - 0.01w_1 + 2.3 > x/y > 27w_2^2 - 1.2w_2 + 2.1$, the fast charging performance and high-temperature ITC performance of the electrochemical apparatus can be simultaneously improved, and gas generation can be suppressed.

In some embodiments, the electrolyte of this application further includes a lithium salt. Instances of the lithium salt may include but are not limited to: inorganic lithium salts such as $LiPF_6$, $LiBF_4$, $LiClO_4$, $LiAlF_4$, $LiSbF_6$, $LiTaF_6$, and $LiWF_7$; lithium tungstates such as $LiWOF_5$; lithium carboxylate salts such as $HCO_2Li$, $CH_3CO_2Li$, $CH_2FCO_2Li$, $CHF_2CO_2Li$, $CF_3CO_2Li$, $CF_3CH_2CO_2Li$, $CF_3CF_2CO_2Li$, $CF_3CF_2CF_2CO_2Li$, and $CF_3CF_2CF_2CF_2CO_2Li$; lithium sulfonates salts such as $FSO_3Li$, $CH_3SO_3Li$, $CH_2FSO_3Li$, $CHF_2SO_3Li$, $CF_3SO_3Li$, $CF_3CF_2SO_3Li$, $CF_3CF_2CF_2SO_3Li$, and $CF_3CF_2CF_2CF_2SO_3Li$; lithium imide salts such as $LiN(FCO)_2$, $LiN(FCO)(FSO_2)$, $LiN(FSO_2)_2$, $LiN(FSO_2)(CF_3SO_2)$, $LiN(CF_3SO_2)_2$, $LiN(C_2F_5SO_2)_2$, lithium cyclic 1,2-perfluoroethane disulfonylimide, lithium cyclic 1,3-perfluoropropane disulfonylimide, and $LiN(CF_3SO_2)(C_4F_9SO_2)$; lithium methide salts such as $LiC(FSO_2)_3$, $LiC(CF_3SO_2)_3$, and $LiC(C_2F_5SO_2)_3$; lithium (malonate) borate salts such as lithium bis(malonate) borate and lithium difluoro(malonate) borate; lithium (malonate) phosphate salts such as lithium tris(malonate) phosphate, lithium difluorobis(malonate) phosphate, and lithium tetrafluoro(malonate) phosphate; fluorine-containing organolithium salts such as $LiPF_4(CF_3)_2$, $LiPF_4(C_2F_5)_2$, $LiPF_4(CF_3SO_2)_2$, $LiPF_4(C_2F_5SO_2)_2$, $LiBF_3CF_3$, $LiBF_3C_2F_5$, $LiBF_3C_3F_7$, $LiBF_2(CF_3)_2$, $LiBF_2(C_2F_5)_2$, $LiBF_2(CF_3SO_2)_2$, and $LiBF_2(C_2F_5SO_2)_2$; lithium oxalatoborate salts such as lithium difluorooxalatoborate and lithium bis(oxalato)borate; and lithium oxalatophosphate salts such as lithium tetrafluorooxalatophosphate, lithium difluorobis(oxalato) phosphate, and lithium tris(oxalato)phosphate.

In some embodiments, the lithium salt includes at least one of the following: $LiPF_6$, $LiBF_4$, $LiAsF_6$, $LiClO_4$, $LiB(C_6H_5)_4$, $LiCH_3SO_3$, $LiCF_3SO_3$, $LiN(SO_2CF_3)_2$, $LiC(SO_2CF_3)_3$, $LiSiF_6$, LiBOB, or LiDFOB.

In some embodiments, based on the total weight of the electrolyte, a percentage of the lithium salt is 0.01 wt% to 20 wt%, 0.01 wt% to 10 wt%, 0.01 wt% to 5 wt%, 0.01 wt% to 3 wt%, 0.1 wt% to 20 wt%, 0.1 wt% to 10 wt%, 0.1 wt% to 5 wt%, 0.1 wt% to 3 wt%, 1 wt% to 20 wt%, 1 wt% to 10 wt%, 1 wt% to 5 wt%, or 1 wt% to 3 wt%.

In some embodiments, the electrolyte further contains any known non-aqueous solvent in the prior art that may be used as a solvent for the electrolyte.

In some embodiments, the non-aqueous solvent includes but is not limited to one or more of the following: cyclic carbonate, linear carbonate, cyclic ether, linear ether, a phosphorus-containing organic solvent, a sulfur-containing organic solvent, and an aromatic fluorine-containing solvent.

In some embodiments, instances of the cyclic carbonate may include but are not limited to one or more of the following: ethylene carbonate (EC), propylene carbonate (PC), and butylene carbonate. In some embodiments, the cyclic carbonate has 3 to 6 carbon atoms.

In some embodiments, instances of the linear carbonate may include but are not limited to one or more of the following: dimethyl carbonate, ethyl methyl carbonate, diethyl carbonate (DEC), methyl n-propyl carbonate, ethyl n-propyl carbonate, and dipropyl carbonate. Instances of the linear carbonate substituted with fluorine may include but are not limited to one or more of the following: bis(fluoromethyl) carbonate, bis(difluoromethyl) carbonate, bis(trifluoromethyl) carbonate, bis(2-fluoroethyl) carbonate, bis(2,2-difluoroethyl) carbonate, bis(2,2,2-trifluoroethyl) carbonate, 2-fluoroethyl methyl carbonate, 2,2-difluoroethyl methyl carbonate, and 2,2,2-trifluoroethyl methyl carbonate.

In some embodiments, instances of the cyclic ether may include but are not limited to one or more of the following: tetrahydrofuran, 2-methyltetrahydrofuran, 1,3-dioxolane, 2-methyl-1,3-dioxolane, 4-methyl-1,3-dioxolane, 1,3-dioxane, 1,4-dioxane, and dimethoxypropane.

In some embodiments, instances of the linear ether may include but are not limited to one or more of the following: dimethoxymethane, 1,1-dimethoxyethane, 1,2-dimethoxyethane, diethoxymethane, 1,1-diethoxyethane, 1,2-diethoxyethane, ethoxymethoxymethane, 1,1-ethoxymethoxyethane, and 1,2-ethoxymethoxyethane.

In some embodiments, instances of the phosphorus-containing organic solvent may include but are not limited to one or more of the following: trimethyl phosphate, triethyl phosphate, dimethyl ethyl phosphate, methyl diethyl phosphate, ethylene methyl phosphate, ethylene ethyl phosphate, triphenyl phosphate, trimethyl phosphite, triethyl phosphite, triphenyl phosphite, tris(2,2,2-trifluoroethyl)phosphate, and tris(2,2,3,3,3-pentafluoropropyl)phosphate.

In some embodiments, instances of the sulfur-containing organic solvent may include but are not limited to one or

more of the following: sulfolane, 2-methylsulfolane, 3-methylsulfolane, dimethyl sulfone, diethyl sulfone, ethyl methyl sulfone, methyl propyl sulfone, dimethyl sulfoxide, methyl methanesulfonate, ethyl methanesulfonate, methyl ethanesulfonate, or ethyl ethanesulfonate. In some embodiments, some hydrogen atoms in the sulfur-containing organic solvent may be substituted with fluorine.

In some embodiments, the aromatic fluorine-containing solvent includes but is not limited to one or more of the following: fluorobenzene, difluorobenzene, trifluorobenzene, tetrafluorobenzene, pentafluorobenzene, hexafluorobenzene, and trifluoromethylbenzene.

## II. Positive electrode

A positive electrode plate includes a positive electrode current collector and a positive electrode active substance layer provided on at least one surface of the positive electrode current collector, where the positive electrode active substance layer contains a positive electrode active substance. There may be one or more positive electrode active substance layers, and each of the plurality of positive electrode active substance layers may contain the same or different positive electrode active substances. The positive electrode active substance is any substance capable of reversibly intercalating and deintercalating metal ions such as lithium ions.

The type of the positive electrode active substance is not particularly limited, provided that metal ions (for example, lithium ions) can be electrochemically absorbed and released. In some embodiments, the positive electrode active substance is a substance that contains lithium and at least one transition metal. Instances of the positive electrode active substance may include but are not limited to lithium transition metal composite oxides and lithium-containing transition metal phosphate compounds.

In some embodiments, transition metals in the lithium transition metal composite oxides include V, Ti, Cr, Mn, Fe, Co, Ni, Cu, and the like. In some embodiments, the lithium transition metal composite oxides include lithium cobalt composite oxides such as $LiCoO_2$, lithium nickel composite oxides such as $LiNiO_2$, lithium manganese composite oxides such as $LiMnO_2$, $LiMn_2O_4$, and $Li_2MnO_4$, and lithium nickel manganese cobalt composite oxides such as $LiNi_{1/3}Mn_{1/3}Co_{1/3}O_2$ and $LiNi_{0.5}Mn_{0.3}Co_{0.2}O_2$, where part of transition metal atoms serving as main bodies of these lithium transition metal composite oxides are substituted with other elements such as Na, K, B, F, Al, Ti, V, Cr, Mn, Fe, Co, Li, Ni, Cu, Zn, Mg, Ga, Zr, Si, Nb, Mo, Sn, and W. Instances of the lithium transition metal composite oxides may include but are not limited to $LiNi_{0.5}Mn_{0.5}O_2$, $LiNi_{0.85}Co_{0.10}Al_{0.05}O_2$, $LiNi_{0.33}Co_{0.33}Mn_{0.33}O_2$, $LiNi_{0.45}Co_{0.10}Al_{0.45}O_2$, $LiMn_{1.8}Al_{0.2}O_4$, and $LiMn_{1.5}Ni_{0.5}O_4$. Instances of combinations of the lithium transition metal composite oxides include but are not limited to a combination of $LiCoO_2$ and $LiMn_2O_4$, where part of Mn in $LiMn_2O_4$ may be substituted with a transition metal (for example, $LiNi_{0.33}Co_{0.33}Mn_{0.33}O_2$), and part of Co in $LiCoO_2$ may be substituted with a transition metal.

In some embodiments, transition metals in the lithium-containing transition metal phosphate compounds include V, Ti, Cr, Mn, Fe, Co, Ni, Cu, and the like. In some embodiments, the lithium-containing transition metal phosphate compounds include iron phosphates such as $LiFePO_4$, $Li_3Fe_2(PO_4)_3$, and $LiFeP_2O_7$, and cobalt phosphates such as $LiCoPO_4$, where part of transition metal atoms serving as main bodies of these lithium-containing transition metal phosphate compounds are substituted with other elements such as Al, Ti, V, Cr, Mn, Fe, Co, Li, Ni, Cu, Zn, Mg, Ga, Zr, Nb, and Si.

A substance with a composition different from that of the positive electrode active substance may be attached to a surface of the positive electrode active substance. Instances of the substance attached to the surface may include but are not limited to: oxides such as aluminum oxide, silicon dioxide, titanium dioxide, zirconium oxide, magnesium oxide, calcium oxide, boron oxide, antimony oxide, and bismuth oxide; sulfates such as lithium sulfate, sodium sulfate, potassium sulfate, magnesium sulfate, calcium sulfate, and aluminum sulfate; carbonates such as lithium carbonate, calcium carbonate, and magnesium carbonate; and carbon. The substance being attached to the surface of the positive electrode active substance can inhibit oxidation reaction of an electrolyte on the surface of the positive electrode active substance and prolong service life of the electrochemical apparatus. When the amount of the substance attached to the surface is too small, the effect cannot be fully exerted. When the amount of the substance attached to the surface is too large, deintercalation and intercalation of lithium ions are hindered, and thus resistance sometimes increases. In this application, the substance attached to the surface of the positive electrode active substance and having the composition different from that of the positive electrode active substance is also referred to as a "positive electrode active substance".

In some embodiments, lithium cobaltate or lithium nickel cobalt manganate is preferably used as the "positive electrode active substance".

In some embodiments, shapes of positive electrode active substance particles include but are not limited to a lumpy shape, a polyhedral shape, a spherical shape, an ellipsoidal shape, a plate shape, a needle shape, and a columnar shape. In some embodiments, the positive electrode active substance particles include primary particles, secondary particles, or combinations thereof. In some embodiments, the primary particles may agglomerate to form the secondary particles.

The positive electrode further includes a positive electrode conductive material, so as to enhance conductivity of

the positive electrode. The positive electrode conductive material is not limited in type, and any known conductive material may be used. Instances of the positive electrode conductive material may include but are not limited to: graphite such as natural graphite and artificial graphite; carbon black such as acetylene black; carbon materials including amorphous carbon such as needle coke; carbon nanotubes; and graphene. The positive electrode conductive material may be used alone or in any combination.

A solvent used for forming a positive electrode slurry is not limited in type, provided that the solvent is capable of dissolving or dispersing the positive electrode active substance, the conductive material, a positive electrode binder, and a thickener used as appropriate to needs. Instances of the solvent used for forming the positive electrode slurry may include any one of an aqueous solvent or an organic solvent. Instances of the aqueous medium may include but are not limited to water and a mixed medium of alcohols and water. Instances of the organic medium may include but are not limited to: aliphatic hydrocarbons such as hexane; aromatic hydrocarbons such as benzene, toluene, xylene, and methylnaphthalene; heterocyclic compounds such as quinoline and pyridine; ketones such as acetone, methyl ethyl ketone, and cyclohexanone; esters such as methyl acetate and methyl acrylate; amines such as diethylenetriamine and N,N-dimethylaminopropylamine; ethers such as diethyl ether, propylene oxide, and tetrahydrofuran (THF); amides such as N-methylpyrrolidone (NMP), dimethylformamide, and dimethylacetamide; and aprotic polar solvents such as hexamethylphosphoramide and dimethyl sulfoxide.

The thickener is typically used to adjust the viscosity of the slurry. In a case that the aqueous medium is used, the thickener and a styrene-butadiene rubber (SBR) emulsion can be used for slurrying. The thickener is not particularly limited in type. Instances of the thickener may include but are not limited to carboxymethyl cellulose, methyl cellulose, hydroxymethyl cellulose, ethyl cellulose, polyvinyl alcohol, oxidized starch, phosphorylated starch, casein, and salts thereof. The foregoing thickener may be used alone or in any combination.

The positive electrode current collector is not particularly limited in type and may be any known material suitable for serving as a positive electrode current collector. Instances of the positive electrode current collector may include but are not limited to: metal materials such as aluminum, stainless steel, nickel coating, titanium, and tantalum; and carbon materials such as carbon cloth and carbon paper. In some embodiments, the positive electrode current collector is a metal material. In some embodiments, the positive electrode current collector is aluminum.

To reduce electronic contact resistance of the positive electrode current collector and the positive electrode active substance layer, a conductive additive may be included in the surface of the positive electrode current collector. Instances of the conductive additive may include but are not limited to carbon and precious metals such as gold, platinum, and silver.

The positive electrode may be prepared by forming the positive electrode active substance layer containing the positive electrode active substance and the binder on the current collector. The positive electrode using the positive electrode active substance can be prepared in a conventional method as follows: The positive electrode active substance, the binder, and the conductive material and the thickener used as appropriate to needs are subjected to dry mixing to prepare a sheet, and then the obtained sheet is pressed onto the positive electrode current collector; or these materials are dissolved or dispersed in a liquid medium to prepare a slurry, and then the obtained slurry is applied onto the positive electrode current collector and drying is performed to form the positive electrode active substance layer on the current collector. In this way, the positive electrode can be obtained.

In some embodiments, based on a total weight of the positive electrode active substance layer, a weight percentage of the positive electrode active substance is M%, where $90 \leq M \leq 99.5$. In some embodiments, $95 \leq M \leq 99$. In some embodiments, M may be 90, 92, 94, 95, 96, 97, 98, or 99, or falls within a range defined by any two of the foregoing values. When the weight percentage of the positive electrode active substance in the positive electrode active substance layer satisfies the foregoing relationship, energy density of the electrochemical apparatus can be significantly increased.

## III. Negative electrode

A negative electrode includes a negative electrode current collector and a negative electrode active substance layer provided on at least one surface of the negative electrode current collector, where the negative electrode active substance layer contains a negative electrode active substance. There may be one or more negative electrode active substance layers, and each of the plurality of negative electrode active substance layers may contain the same or different negative electrode active substances. The negative electrode active substance is any substance capable of reversibly intercalating and deintercalating metal ions such as lithium ions. In some embodiments, a rechargeable capacity of the negative electrode active substance is greater than a discharge capacity of the positive electrode active substance to prevent lithium metal from unexpectedly precipitating at the negative electrode during charging.

Any well-known current collector may be used as a current collector for holding the negative electrode active substance. Instances of the negative electrode current collector include but are not limited to metal materials such as aluminum, copper, nickel, stainless steel, and nickel-plated steel. In some embodiments, the negative electrode current collector is copper.

In a case that the negative electrode current collector is a metal material, the negative electrode current collector may take forms including but not limited to a metal foil, a metal cylinder, a metal coil, a metal plate, a metal film, a sheet metal mesh, a punched metal, and a foamed metal. In some embodiments, the negative electrode current collector is a metal film. In some embodiments, the negative electrode current collector is a copper foil. In some embodiments, the negative electrode current collector is a rolled copper foil based on a rolling method or an electrolytic copper foil based on an electrolytic method.

In some embodiments, a thickness of the negative electrode current collector is greater than 1 μm or greater than 5 μm. In some embodiments, the thickness of the negative electrode current collector is less than 100 μm or less than 50 μm. In some embodiments, the thickness of the negative electrode current collector falls within a range defined by any two of the foregoing values.

The negative electrode active substance is not particularly limited, provided that it can reversibly absorb and release lithium ions. Instances of the negative electrode active substance may include but are not limited to: carbon materials such as natural graphite and artificial graphite; metals such as silicon (Si) and tin (Sn); and oxides of metal elements such as Si and Sn. The negative electrode active substance may be used alone or in combination.

The negative electrode active substance layer may further include a negative electrode binder. The negative electrode binder can improve binding between particles of the negative electrode active substance and binding between the negative electrode active substance and the current collector. The negative electrode binder is not particularly limited in type, provided that its material is stable to the electrolyte or a solvent used in manufacturing of the electrode. In some embodiments, the negative electrode binder includes a resin binder. Instances of the resin binder include but are not limited to fluororesins, polyacrylonitrile (PAN), polyimide resins, acrylic resins, and polyolefin resins. When an aqueous solvent is used to prepare a negative electrode mixture slurry, the negative electrode binder includes but is not limited to carboxymethyl cellulose (CMC) or its salt, styrene-butadiene rubber (SBR), polyacrylic acid (PAA) or its salt, and polyvinyl alcohol.

The negative electrode may be prepared in the following method: The negative electrode mixture slurry containing the negative electrode active substance, the resin binder, and the like is applied onto the negative electrode current collector. After drying, rolling is performed to form the negative electrode active substance layer on two surfaces of the negative electrode current collector, to obtain the negative electrode.

## IV. Separator

In order to prevent short circuit, a separator is typically provided between the positive electrode and the negative electrode. In this case, the electrolyte of this application typically permeates the separator for use.

The separator is not particularly limited in material and shape, provided that the separator does not significantly impair the effects of this application. The separator may be a resin, glass fiber, an inorganic substance, or the like that is formed of a material stable to the electrolyte of this application. In some embodiments, the separator includes a porous sheet or nonwoven fabric-like substance having an excellent electrolyte retention property. Instances of the material of the resin or glass fiber separator may include but are not limited to polyolefin, aromatic polyamide, polytetrafluoroethylene, and polyethersulfone. In some embodiments, the polyolefin is polyethylene or polypropylene. In some embodiments, the polyolefin is polypropylene. The material of the separator may be used alone or in any combination.

The separator may alternatively be a material formed by laminating the foregoing materials, and instances thereof include but are not limited to a three-layer separator formed by laminating polypropylene, polyethylene, and polypropylene in order.

Instances of the material of the inorganic substance may include but are not limited to: oxides such as aluminum oxide and silicon dioxide; nitrides such as aluminum nitride and silicon nitride; and sulfates (for example, barium sulfate and calcium sulfate). The form of the inorganic substance may include but is not limited to a granular or fibrous form.

The form of the separator may be a thin-film form, and instances thereof include but are not limited to a non-woven fabric, a woven fabric, and a microporous film. In the thin-film form, the separator has a pore diameter of 0.01 μm to 1 μm and a thickness of 5 μm to 50 μm. In addition to the independent thin-film-like separator, the following separator may alternatively be used: a separator that is obtained by using a resin-based binder to form a composite porous layer containing inorganic particles on the surface of the positive electrode and/or the negative electrode, for example, a separator that is obtained by using fluororesin as a binder to form a porous layer on two surfaces of the positive electrode with alumina particles of which 90% have a particle size less than 1 μm.

The thickness of the separator is random. In some embodiments, the thickness of the separator is greater than 1 μm, greater than 5 μm, or greater than 8 μm. In some embodiments, the thickness of the separator is less than 50 μm, less than 40 μm, or less than 30 μm. In some embodiments, the thickness of the separator falls within a range defined by any two of the foregoing values. When the thickness of the separator falls within the foregoing range, its insulation performance and mechanical strength can be ensured, and the rate performance and energy density of the electrochemical apparatus can be ensured.

When a porous material such as a porous sheet or a nonwoven fabric is used as the separator, the porosity of the separator is random. In some embodiments, the porosity of the separator is greater than 10%, greater than 15%, or greater than 20%. In some embodiments, the porosity of the separator is less than 60%, less than 50%, or less than 45%. In some embodiments, the porosity of the separator falls within a range defined by any two of the foregoing values. When the porosity of the separator falls within the foregoing range, its insulation performance and the mechanical strength can be ensured and film resistance can be suppressed, so that the electrochemical apparatus has good safety performance.

The average pore diameter of the separator is also random. In some embodiments, the average pore diameter of the separator is less than 0.5 μm or less than 0.2 μm. In some embodiments, the average pore diameter of the separator is greater than 0.05 μm. In some embodiments, the average pore diameter of the separator falls within a range defined by any two of the foregoing values. If the average pore diameter of the separator exceeds the foregoing range, a short circuit is likely to occur. When the average pore diameter of the separator falls within the foregoing range, the electrochemical apparatus has good safety performance.

## V. Electrochemical apparatus

The electrochemical apparatus of this application includes any apparatus in which an electrochemical reaction takes place. Specific instances of the apparatus include lithium metal secondary batteries or lithium-ion secondary batteries. The electrochemical apparatus of this application includes the electrolyte described in the foregoing embodiments. In some embodiments, the electrochemical apparatus of this application further includes a positive electrode, a negative electrode, and a separator sandwiched between the positive electrode and the negative electrode.

In view of design room reserved for an electrochemical apparatus in a notebook computer, the electrochemical apparatus typically tends to be narrow and long in shape. Due to uneven current distribution and unevenly formed SEI film on a narrow and long battery, lithium precipitation is more likely to occur at upper and lower edges in normal-temperature or low-temperature charging, leading to degradation of capacity. In addition, precipitated lithium dendrites may also pierce the separator sandwiched between the positive electrode and the negative electrode to cause a short circuit in the battery, leading to safety problems.

In some embodiments, the positive electrode, the negative electrode, and the separator are wound to form a cell. In some embodiments, the positive electrode, the negative electrode, and the separator are stacked to form a cell.

It is found in this application that adjusting a size of the cell can improve a charging rate window of the electrochemical apparatus and alleviate lithium precipitation, thereby improving fast charging performance of the electrochemical apparatus.

In some embodiments, a length L and a width W of the cell satisfy 20 mm $\leq$ L $\leq$ 300 mm, 20 mm $\leq$ W $\leq$ 100 mm, and 1 $\leq$ L/W $\leq$ 4. In some embodiments, the length L of the cell may be but is not limited to 20 mm, 50 mm, 75 mm, 100 mm, 125 mm, 150 mm, 175 mm, 200 mm, 225 mm, 250 mm, 275 mm, or 300 mm, or falls within a range defined by any two of the foregoing values. In some embodiments, the width W of the cell may be but is not limited to 20 mm, 30 mm, 40 mm, 50 mm, 60 mm, 70 mm, 80 mm, 90 mm, or 100 mm, or falls within a range defined by any two of the foregoing values. In some embodiments, a length-to-width ratio L/W of the cell may be but is not limited to 1, 2, 3, or 4, or falls within a range defined by any two of the foregoing values.

In some embodiments, a thickness T of the cell satisfies 2 mm $\leq$ T $\leq$ 12 mm. In some embodiments, the thickness T of the cell may be but is not limited to 2, 4, 6, 8, 10, or 12, or falls within a range defined by any two of the foregoing values. In some embodiments, a width-to-thickness ratio W/T of the cell satisfies W/T $\geq$ 5. In some embodiments, the width-to-thickness ratio W/T of the cell satisfies W/T $\leq$ 25. In some embodiments, W/T may be but is not limited to 5, 10, 15, 20, or 25, or falls within a range defined by any two of the foregoing values.

In some embodiments, designing a size of the battery and simultaneously optimizing the percentage of the carboxylate compound in the electrolyte can further improve the charging rate window of the electrochemical apparatus and alleviate lithium precipitation, thereby improving the fast charging performance of the electrochemical apparatus. In some embodiments, the length L and the width W of the cell and the percentage $w_1$ of the carboxylate compound represented by formula (I) satisfy $w_1 \times 100/(L/W) \geq 10$. In some embodiments, the length L and the width W of the cell and the percentage $w_1$ of the carboxylate compound represented by formula (I) satisfy $w_1 \times 100/(L/W) \leq 40$. In some embodiments, $w_1 \times 100/(L/W)$ may be but is not limited to 10, 15, 20, 25, 30, 35, or 40, or falls within a range defined by any two of the foregoing values.

## VI. Electronic apparatus

This application further provides an electronic apparatus, including the electrochemical apparatus according to this application.

The electrochemical apparatus of this application is not particularly limited to any purpose, and may be used in any electronic apparatus known in the prior art. In some embodiments, the electrochemical apparatus of this application

may be used without limitation in notebook computers, pen-input computers, mobile computers, electronic book players, portable telephones, portable fax machines, portable copiers, portable printers, stereo headsets, video recorders, liquid crystal display televisions, portable cleaners, portable CD players, mini-disc players, transceivers, electronic notebooks, calculators, storage cards, portable recorders, radios, backup power sources, motors, automobiles, motorcycles, motor bicycles, bicycles, lighting appliances, toys, game machines, clocks, electric tools, flash lamps, cameras, large household batteries, and lithium-ion capacitors.

The following uses a lithium-ion battery as an example and describes preparation of a lithium-ion battery with reference to specific examples. Persons skilled in the art understand that the preparation method described in this application is only an example, and that all other suitable preparation methods fall within the scope of this application.

**(I). Preparation of lithium-ion battery**

(1) Preparation of positive electrode

A positive electrode active material lithium cobalt oxide ($LiCoO_2$), a conductive agent Super P, and a binder polyvinylidene fluoride were mixed at a weight ratio of 97:1:2. N-methylpyrrolidone (NMP) was added. Then, the resulting mixture was stirred to uniformity under the action of a vacuum mixer to obtain a positive electrode slurry. Then, the positive electrode slurry was evenly applied onto a positive electrode current collector aluminum foil. The aluminum foil was dried, followed by cold pressing, cutting, and slitting. Then, drying was performed in vacuum to obtain a positive electrode plate.

(2) Preparation of negative electrode

A negative electrode active material artificial graphite, a thickener sodium carboxymethylcellulose (CMC), and a binder styrene-butadiene rubber (SBR) were mixed at a weight ratio of 97:1:2. Deionized water was added. Then, the resulting mixture was stirred under the action of a vacuum mixer to obtain a negative electrode slurry. Then, the negative electrode slurry was evenly applied onto a negative electrode current collector copper foil. The copper foil was dried, followed by cold pressing, cutting, and slitting. Then, drying was performed in vacuum to obtain a negative electrode plate.

(3) Preparation of electrolyte

In a dry argon atmosphere glove box, ethylene carbonate (EC), ethylene carbonate (PC), and diethyl carbonate (DEC) were mixed at a weight ratio of 1:3:6, and a lithium salt $LiPF_6$ was then added. The resulting mixture was mixed to uniformity to form a base electrolyte, where the concentration of $LiPF_6$ was 1.2 mol/L. Additives were added into the base electrolyte based on needs to obtain electrolytes in examples and comparative examples of this application, where for the specific types and percentages of the additives, reference may be made to the following tables 1-3.

4. Preparation of separator

Boehmite and polyacrylate were mixed, and the resulting mixture was dissolved in deionized water to form a coating slurry. Then, the coating slurry was evenly applied onto two surfaces of a polyethylene porous substrate by using a micro-gravure coating method, and dried to obtain the separator.

(5) Preparation of lithium-ion battery

The positive electrode, the separator, and the negative electrode were stacked in order, where the separator was sandwiched between the positive electrode and the negative electrode for separation. Then, the resulting stack was wound to obtain a cell. After tabs were welded, the cell was placed in an outer packaging aluminum foil film, with an injection opening left. The prepared electrolyte was injected into a dried cell through the injection opening, followed by processes such as vacuum packaging, standing, formation, shaping, and capacity testing, to obtain a lithium-ion battery.

**(II). Test method**

1. Test method for compositions and percentages thereof in electrolyte

The battery was discharged to 2.8 V at a constant current of 0.1C, and the weight of the battery was weighed and denoted as m. Then, the battery was disassembled, the cell and the outer packaging aluminum foil film disassembled from the battery were rapidly placed in high-purity acetonitrile (the purity was greater than or equal to 99.9%) for extraction. Then, the extracted supernatant was subjected to a gas chromatography test to obtain compositions and relative

percentages p thereof in the electrolyte. The extracted cell and outer packaging aluminum foil film were dried in a vacuum oven. A total weight was weighed and denoted as m', so the weight n of the electrolyte satisfies n = m - m', and the weights of the compositions in the battery were products of n and the relative percentages p of the compositions.

2. Calculation method for cyano group enrichment

Cyano group enrichment was calculated through the following formula:

$$\text{Cyano group enrichment} = \frac{\text{x (total molar mass of cyano groups)}}{\text{y (total molar mass of nitrile compounds)}}$$

$$= \frac{\frac{n1}{M1}\times\varepsilon 1+\frac{n2}{M2}\times\varepsilon 2+\cdots+\frac{nx}{Mx}\times\varepsilon x}{\frac{n1}{M1}+\frac{n2}{M2}+\cdots+\frac{nx}{Mx}} = \frac{\frac{p1}{M1}\times\varepsilon 1+\frac{p2}{M2}\times\varepsilon 2+\cdots+\frac{px}{Mx}\times\varepsilon x}{\frac{p1}{M1}+\frac{p2}{M2}+\cdots+\frac{px}{Mx}}$$

n1, n2, ..., and nx represent masses (g) of different nitrile compounds;
p1, p2, ..., and px represent relative percentages (%) of different nitrile compounds;
M1, M2, ..., and Mx represent relative molar masses (g/mol) of different nitrile compounds; and
$\varepsilon 1$, $\varepsilon 2$, ..., and $\varepsilon x$ represent numbers of cyano groups in different nitrile compounds.

3. Cell size test method

The outer packaging aluminum foil film was disassembled and removed to obtain a cell. As shown in FIG 4, a length L, a width W, and a thickness T of the cell were measured with a micrometer respectively.

4. Fast charging performance test for lithium-ion battery

Lithium-ion batteries were divided into three groups and subjected to charge and discharge cycle test at currents of 0.7C, 1.5C, and 3C respectively, where there were 10 lithium-ion batteries in each group.

The lithium-ion batteries were left standing for 30 minutes at 25°C, charged to a voltage of 4.5 V at 0.7C, 1.5C, and 3C respectively, charged to 0.05C at a constant voltage, and discharged to 3.0 V at constant currents of 0.7C, 1.5C, and 3C respectively. 250 charge and discharge cycles were performed according to the foregoing procedures.

First-cycle discharge capacities of the lithium-ion batteries were recorded, and an average of the first-cycle discharge capacities of the batteries in the groups was measured and denoted as C1; and discharge capacities of the lithium-ion batteries after 250 cycles were recorded, and an average of the discharge capacities of the batteries in the groups was measured and denoted as C250. A cycling capacity retention rate of the lithium-ion battery at 0.7C, 1.5C, and 3C was calculated by using the following formula:

$$\text{Cycling capacity retention rate} = (C250/C1) \times 100\%.$$

5. High-temperature interval cycle (ITC) test for lithium-ion battery

The lithium-ion battery was placed in a 45°C thermostat and left standing for 30 minutes. Then, the lithium-ion battery was discharged to 3.0 V at a current of 0.5C, charged to 4.5 V at a current of 1C, and left standing for 24 hours. This was one charge and discharge cycle. 50 charge and discharge cycles were performed according to the foregoing procedures. A first-cycle discharge capacity and a discharge capacity after 50 cycles were recorded as C and C' respectively. A cycling capacity retention rate of the lithium-ion battery was calculated by using the following formula:

$$\text{Cycling capacity retention rate} = (C'/C) \times 100\%.$$

In addition, an initial thickness $\eta$ and a thickness $\eta'$ after 50 cycles of the lithium-ion battery were measured with a micrometer respectively. A thickness growth rate of the lithium-ion battery was calculated by using the following formula:

$$\text{Thickness growth rate} = (\eta'/\eta - 1) \times 100\%.$$

**(III). Test results**

Table 1 shows the effect of percentages of the carboxylate compound and FEC in the electrolyte and a ratio thereof on fast charging performance and high-temperature ITC performance of the lithium-ion battery.

**Table 1**

| | Carboxylate compound | | FEC | $w_1/w_2$ | Capacity retention rate after 250 cycles at 4.5 V at 25°C | | | After 50 ITC cycles at 4.5 V at 45°C | |
|---|---|---|---|---|---|---|---|---|---|
| | Type | Percentage/$w_1$ | Percentage/$w_2$ | | 0.7C | 1.5C | 3C | Capacity retention rate | Thickness growth rate |
| Comparative Example 1-1 | / | / | 1.0% | / | 61% | 50% | 33% | 44% | 85% |
| Comparative Example 1-2 | / | / | 5.0% | / | 66% | 51% | 35% | 45% | 89% |
| Comparative Example 1-3 | / | / | 13.0% | / | 68% | 49% | 30% | 43% | 122% |
| Comparative Example 1-4 | Ethyl acetate | 20% | 1.0% | 20.0 | 62% | 58% | 55% | 41% | 93% |
| Comparative Example 1-5 | Ethyl propionate | 20% | 1.0% | 20.0 | 65% | 61% | 54% | 44% | 91% |
| Comparative Example 1-6 | Propyl propionate | 20% | 1.0% | 20.0 | 64% | 60% | 50% | 46% | 84% |
| Comparative Example 1-7 | Ethyl propionate | 3% | 5.0% | 0.6 | 67% | 53% | 35% | 46% | 90% |
| Comparative Example 1-8 | Ethyl propionate | 5% | 5.0% | 1.0 | 68% | 64% | 36% | 47% | 91% |
| Example 1-1 | Ethyl propionate | 10% | 5.0% | 2.0 | 71% | 67% | 40% | 48% | 94% |
| Example 1-2 | Ethyl propionate | 20% | 5.0% | 4.0 | 73% | 69% | 52% | 50% | 96% |
| Example 1-3 | Ethyl propionate | 40% | 5.0% | 8.0 | 75% | 71% | 55% | 47% | 102% |
| Example 1-4 | Ethyl propionate | 60% | 5.0% | 12.0 | 72% | 68% | 55% | 44% | 113% |
| Comparative Example 1-9 | Ethyl propionate | 70% | 3.5% | 20.0 | 69% | 65% | 47% | 40% | 88% |
| Example 1-5 | Ethyl propionate | 20% | 2.0% | 10.0 | 66% | 62% | 56% | 46% | 70% |
| Example 1-6 | Ethyl propionate | 20% | 4.0% | 5.0 | 70% | 66% | 55% | 47% | 72% |
| Comparative Example 1-10 | Ethyl acetate | 10% | 6.0% | 1.7 | 68% | 64% | 48% | 45% | 97% |
| Example 1-7 | Ethyl acetate | 20% | 5.0% | 4.0 | 75% | 71% | 56% | 45% | 89% |

(continued)

| | Carboxylate compound | | FEC | $w_1/w_2$ | Capacity retention rate after 250 cycles at 4.5 V at 25°C | | | After 50 ITC cycles at 4.5 V at 45°C | |
|---|---|---|---|---|---|---|---|---|---|
| | Type | Percentage/$w_1$ | Percentage/$w_2$ | | 0.7C | 1.5C | 3C | Capacity retention rate | Thickness growth rate |
| Example 1-8 | Ethyl acetate | 40% | 4.0% | 10.0 | 75% | 65% | 56% | 44% | 88% |
| Example 1-9 | Propyl propionate | 20% | 6.0% | 3.3 | 72% | 62% | 48% | 50% | 67% |
| Example 1-10 | Propyl propionate | 40% | 5.0% | 8.0 | 73% | 63% | 50% | 47% | 69% |
| Example 1-11 | Propyl propionate | 60% | 3.0% | 20.0 | 70% | 64% | (%) | 44% | 76% |
| Example 1-12 | Ethyl acetate + ethyl propionate | 10% + 10% | 5.0% | 4.0 | 74% | 64% | 52% | 45% | 92% |
| Example 1-13 | Ethyl propionate + propyl propionate | 10% + 10% | 5.0% | 4.0 | 73% | 63% | 52% | 48% | 89% |
| Example 1-14 | Ethyl propionate + propyl propionate | 25% + 15% | 5.0% | 8.0 | 76% | 66% | 55% | 46% | 72% |
| Example 1-15 | Ethyl acetate + ethyl propionate + propyl propionate | 10% + 20% + 10% | 5.0% | 8.0 | 74% | 64% | 53% | 47% | 89% |
| Note: The symbol "/" indicates that there is no corresponding composition. | | | | | | | | | |

The electrochemical test results in Table 1 indicate that adding the carboxylate compound and FEC into the electrolyte and adjusting the percentages and ratio thereof to satisfy $5\% \leq w_1 \leq 60\%$, $2\% \leq w_2 \leq 12\%$, and $2 \leq w_1/w_2 \leq 20$ can improve the fast charging performance of the electrochemical apparatus without deteriorating or slightly deteriorating the high-temperature ITC performance of the electrochemical apparatus.

Comparative Examples 1-1 to 1-3 show that adding FEC alone into the electrolyte and keeping the percentage thereof within an appropriate range can improve the fast charging performance of the electrochemical apparatus to some extent. However, when the percentage of FEC is excessively high (for example, greater than 12%), gas generation is aggravated, leading to significant growth in the thickness of the battery in high-temperature interval cycles. In addition, the excessively high percentage of FEC does not improve but rather deteriorates the fast charging performance of the electrochemical apparatus at high currents (for example, 1.5C and 3C). This is because an appropriate amount of FEC can effectively alleviate damage to the SEI film at the negative electrode in charging and discharging of the battery and repair the damaged SEI film in a timely manner, thereby improving overcharge performance. However, when the percentage of FEC is excessively high, an excessively thick SEI film is easily formed, and intensification of the film formation reaction further causes lithium precipitation on the cell, leading to rapid degradation of the battery capacity. In addition, when the percentage of FEC is excessively high, the positive electrode structure is damaged under the ITC intermittent cycle condition, and the oxygen release reaction occurs to oxidize FEC to generate $CO_2$, leading to swelling and gas generation of the battery.

As compared with Comparative Example 1-1, in Comparative Examples 1-4 to 1-6, the linear carboxylate compound is further added into the electrolyte, which improves the fast charging performance of the electrochemical apparatus, and especially significantly improves the fast charging performance at high currents (for example, 1.5C and

3C). Compared with ethyl acetate, ethyl propionate and propyl propionate almost do not deteriorate the high-temperature ITC performance while improving the fast charging performance, and may even improve the high-temperature ITC performance.

From comparison between Examples 1-1 to 1-4 and Comparative Examples 1-5 and 1-7 to 1-9, it can be learned that when the percentages and ratio of the carboxylate compound and FEC satisfy 5% $\leq w_1 \leq$ 60%, 2% $\leq w_2 \leq$ 12%, and 2 $\leq w_1/w_2 \leq$ 20, the fast charging performance of the electrochemical apparatus can be improved without deteriorating or slightly deteriorating the high-temperature ITC performance of the electrochemical apparatus. It can be learned from comparison between Examples 1-5 and 1-6 and Comparative Example 1-5 that further adjusting $w_1/w_2$ to be within a range of 4 $\leq w_1/w_2 \leq$ 10 can not only improve the fast charging performance of the electrochemical apparatus but also simultaneously improve the high-temperature ITC performance of the electrochemical apparatus. From comparison between Examples 1-7 and 1-8 and Comparative Examples 1-4 and 1-10 or comparison between Examples 1-9 to 1-11 and Comparative Example 1-6, it can be learned that the same conclusion can also be obtained.

In Examples 1-12 to 1-15, combinations of a variety of carboxylate compounds are used, and when 5% $\leq w_1 \leq$ 60%, 2% $\leq w_2 \leq$ 12%, and 2 $\leq w_1/w_2 \leq$ 20 are satisfied, the fast charging performance and the high-temperature ITC performance are expected to be simultaneously improved. For example, both propyl propionate and ethyl propionate being added into the electrolyte can improve the fast charging performance more efficiently while maintaining excellent high-temperature ITC performance.

Table 2 shows the effect of the cyano group enrichment and percentages of carboxylate and FEC in the electrolyte on the fast charging performance and high-temperature ITC performance of the lithium-ion battery, where the examples in Table 2 are improvements made based on Example 1-14, with a difference being specifically addition of a nitrile additive to the electrolyte.

The performance test results in Table 2 indicate that further adding an appropriate amount of nitrile additive into the electrolyte can improve the high-temperature ITC performance of the electrochemical apparatus without deteriorating or slightly deteriorating the fast charging performance of the electrochemical apparatus.

As compared with Example 1-14, in Examples 2-1 to 2-8, a single nitrile additive is used in the electrolyte, which slightly deteriorates the fast charging performance of the electrochemical apparatus but significantly improves the high-temperature ITC performance. However, when the single nitrile compound is added in excess, the viscosity of the electrolyte is increased to some extent, which causes untimely intercalation and deintercalation reactions of lithium ions, and in turn leads to lithium precipitation, thus adversely affecting the high-temperature ITC performance to some extent.

As compared with Example 1-14, in Examples 2-9 to 2-29, a variety of nitrile additives are introduced into the electrolyte. Referring to Examples 2-9 to 2-17, in a case that a specified amount of nitrile compound is added, when the cyano group enrichment x/y satisfies 2.16 $\leq$ x/y $\leq$ 2.71, the high-temperature ITC performance of the electrochemical apparatus can be effectively improved while the fast charging performance of the electrochemical apparatus is substantially not deteriorated.

It can be further learned from data in Table 2 that when the cyano group enrichment x/y satisfies 2.16 $\leq$ x/y $\leq$ 2.71 and the percentage $w_3$ of the nitrile compound is controlled to be within a range of 0.1% $\leq w_3 \leq$ 12%, the deterioration of the fast charging performance caused by the single nitrile can be effectively alleviated while the improvement effect of the addition of the single nitrile on the high-temperature ITC performance can be broken.

In addition, Examples 2-26 to 2-29 indicate that the total percentage of the nitrile should not be excessively high or excessively low on the promise of satisfying the foregoing range of the cyano group enrichment. However, it can be learned from comparison between Examples 2-26 to 2-29 and Examples 2-19 and 2-25 that when the cyano group enrichment x/y satisfies 2.16 $\leq$ x/y $\leq$ 2.71, an upper limit of the use amount of the single nitrile can be broken, and the high-temperature ITC performance can be improved to the maximum extent.

**Table 2**

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 1-14 | / | / | / | / | / | / | / | / | / | / | / | / | 76% | 66% | 55% | 46% | 72% |
| Example 2-1 | Butanedinitrile | 0.5% | / | / | / | / | / | / | / | / | 0.5% | 2.00 | 75% | 65% | 55% | 47% | 62% |
| Example 2-2 | Butanedinitrile | 3.0% | / | / | / | / | / | / | / | / | 3.0% | 2.00 | 75% | 64% | 54% | 51% | 58% |
| Example 2-3 | Butanedinitrile | 6.0% | / | / | / | / | / | / | / | / | 6.0% | 2.00 | 73% | 63% | 52% | 48% | 60% |
| Example 2-4 | Butanedinitrile | 8.0% | / | / | / | / | / | / | / | / | 8.0% | 2.00 | 69% | 63% | 50% | 43% | 66% |
| Example 2-5 | 1,3,6-hexanetric-arbonitrile | 0.5% | / | / | / | / | / | / | / | / | 0.5% | 3.00 | 76% | 63% | 55% | 48% | 57% |
| Example 2- | 1,3,6-hexanetric-arbonitrile | 3.0% | / | / | / | / | / | / | / | / | 3.0% | 3.00 | 72% | 58% | 51% | 54% | 51% |
| Example 2-7 | 1,3,6-hexanetric-arbonitrile | 6.0% | / | / | / | / | / | / | / | / | 6.0% | 3.00 | 71% | 57% | 50% | 52% | 50% |
| Example 2-8 | 1,3,6-hexanetric-arbonitrile | 8.0% | / | / | / | / | / | / | / | / | 8.0% | 3.00 | 70% | 57% | 49% | 51% | 49% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-9 | Butanedinitrile | 1.0% | 1,3,6-hexanetricarbonitrile | 5.0% | / | / | / | / | / | / | 6.0% | 2.71 | 74% | 62% | 52% | 47% | 46% |
| Example 2-10 | Butanedinitrile | 3.0% | 1,3,6-hexanetricarbonitrile | 3.0% | / | / | / | / | / | / | 6.0% | 2.33 | 75% | 63% | 53% | 58% | 35% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-11 | Butanedinitrile | 4.3% | 1,3,6-hexanetricarbonitrile | 1.7% | / | / | / | / | / | / | 6.0% | 2.16 | 76% | 64% | 55% | 53% | 40% |
| Example 2-12 | Butanedinitrile | 0.2% | 1,3,6-hexanetricarbonitrile | 4.8% | Glutaronitrile | 1.0% | / | / | / | / | 6.0% | 2.72 | 73% | 60% | 50% | 49% | 44% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-13 | Butanedinitrile | 1.5% | 1,3,6-hexanetricarbonitrile | 3.5% | Glutaronitrile | 1.0% | / | / | / | / | 6.0% | 2.44 | 74% | 65% | 53% | 60% | 33% |
| Example 2-14 | Butanedinitrile | 3.4% | 1,3,6-hexanetricarbonitrile | 1.6% | Glutaronitrile | 1.0% | / | / | / | / | 6.0% | 2.16 | 76% | 66% | 55% | 55% | 38% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-15 | Butanedinitrile | 0.2% | 1,3,6-hexanetricarbonitrile | 4.7% | Glutaronitrile | 0.6% | Ethylene glycol bis(propanenitrile) ether | 0.5% | / | / | 6.0% | 2.73 | 72% | 61% | 51% | 51% | 42% |
| Example 2-16 | Butanedinitrile | 1.5% | 1,3,6-hexanetricarbonitrile | 2.5% | Glutaronitrile | 1.0% | Ethylene glycol bis(propanenitrile) ether | 1.0% | / | / | 6.0% | 2.31 | 75% | 65% | 54% | 62% | 31% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-17 | Butanedinitrile | 1.5% | 1,3,6-hexanetricarbonitrile | 1.3% | Glutaronitrile | 1.0% | Ethylene glycol bis(propanenitrile) ether | 2.2% | / | / | 6.0% | 2.16 | 76% | 66% | 55% | 60% | 33% |
| Example 2-18 | Butanedinitrile | 1.0% | 1,3,6-hexanetricarbonitrile | 4.0% | / | / | / | / | / | / | 5.0% | 2.67 | 73% | 63% | 53% | 46% | 49% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-19 | Butanedinitrile | 4.3% | 1,3,6-hexanetricarbonitrile | 3.7% | / | / | / | / | / | / | 8.0% | 2.30 | 74% | 64% | 54% | 55% | 34% |
| Example 2-20 | Butanedinitrile | 0.2% | 1,3,6-hexanetricarbonitrile | 4.3% | Glutaronitrile | 1.0% | / | / | / | / | 5.5% | 2.69 | 73% | 63% | 53% | 52% | 43% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-21 | Butanedinitrile | 3.4% | 1,3,6-hexanetricarbonitrile | 3.1% | Glutaronitrile | 1.0% | / | / | / | / | 7.5% | 2.27 | 73% | 64% | 55% | 57% | 32% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-22 | 1,3,6-hexanetric arbonitrile | 3.6% | Ethylene glycol bis(propanenitrile) ether | 0.4% | Glutaronitrile | 1.0% | / | / | / | / | 5.0% | 2.66 | 72% | 62% | 51% | 56% | 40% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-23 | Butanedinitrile | 1.5% | 1,3,6-hexanetricarbonitrile | 2.3% | Glutaronitrile | 1.0% | Ethylene glycol bis(propanenitrile) ether | 2.2% | / | / | 7.0% | 2.26 | 74% | 64% | 55% | 58% | 31% |
| Example 2-24 | Butanedinitrile | 1.5% | 1,3,6-hexanetricarbonitrile | 1.0% | Glutaronitrile | 1.0% | 1,2,3-tris(2-cyanoethoxy)propane | 2.5% | / | / | 6.0% | 2.37 | 74% | 64% | 53% | 63% | 30% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-25 | Butanedinitrile | 1.5% | 1,3,6-hexanetricarbonitrile | 1.0% | Glutaronitrile | 1.0% | Ethylene glycol bis(propanenitrile) ether | 2.1% | 1,2,3-tris(2-cyanoethoxy)propane | 2.4% | 8.0% | 2.28 | 75% | 65% | 55% | 61% | 29% |
| Example 2-26 | Butanedinitrile | 0.2% | 1,3,6-hexanetricarbonitrile | 0.3% | / | / | / | / | / | / | 0.5% | 2.43 | 76% | 65% | 53% | 46% | 72% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-27 | Butanedinitrile | 6.0% | 1,3,6-hexanetricarbonitrile | 7.0% | / | / | / | / | / | / | 13.0% | 2.40 | 70% | 60% | 50% | 41% | 68% |
| Example 2-28 | Butanedinitrile | 0.1% | 1,3,6-hexanetricarbonitrile | 0.2% | Glutaronitrile | 0.1% | Ethylene glycol bis(propanenitrile) ether | 0.1% | / | / | 0.5% | 2.31 | 76% | 65% | 54% | 51% | 56% |

| | Nitrile additive | | | | | | | | | | | | Capacity retention rate after 250 cycles at 4.5 V at 25°C | | | After 50 ITC cycles at 4.5 V at 45°C | |
|---|---|---|---|---|---|---|---|---|---|---|---|---|---|---|---|---|---|
| | Type I | Percentage p1 | Type II | Percentage p2 | Type III | Percentage p3 | Type IV | Percentage p4 | Type V | Percentage p5 | Total percentage | Cyano group enrichment | 0.7C | 1.5C | 3C | Capacity retention rate | Thickness growth rate |
| Example 2-29 | Butanedinitrile | 3.0% | 1,3,6-hexanetricarbonitrile | 4.0% | Glutaronitrile | 3.0% | Ethylene glycol bis(propanenitrile) ether | 3.0% | / | / | 13.0% | 2.23 | 71% | 62% | 52% | 43% | 66% |

Note: The symbol "/" indicates that there is no corresponding composition.

Table 3 shows the effect of the cell size and the percentage of the carboxylate compound in the electrolyte on the high-temperature ITC performance of the lithium-ion battery, where the examples in Table 3 are improvements made based on Example 2-23, with a difference being specifically the difference of the cell size and/or the percentage of the carboxylate compound in the electrolyte.

**Table 3**

| | Carboxylate | | Cell size (mm) | | | Length-to-width ratio (L/W) | Width-to-thickness ratio (W/T) | $w_1 \times 100/(L/W)$ | After 250 cycles at 4.5 V at 25°C at different charging rates | | |
|---|---|---|---|---|---|---|---|---|---|---|---|
| | Type | Percentage $w_1$ | Length L | Width W | Thickness T | | | | 0.7C | 1.5C | 3C |
| Example 2-23 | Ethyl propionate + propyl propionate | 25% + 15% | 100 | 50 | 4 | 2 | 12.5 | 20 | 75% | 65% | 55% |
| Example 3-1 | Ethyl propionate + propyl propionate | 25% + 15% | 70 | 50 | 4 | 1.4 | 12.5 | 28.6 | 76% | 65% | 55% |
| Example 3-2 | Ethyl propionate + propyl propionate | 25% + 15% | 75 | 50 | 4 | 1.5 | 12.5 | 26.7 | 75% | 65% | 55% |
| Example 3-3 | Ethyl propionate + propyl propionate | 25% + 15% | 150 | 50 | 4 | 3 | 12.5 | 13.3 | 74% | 63% | 50% |
| Example 3-4 | Ethyl propionate + propyl propionate | 25% + 15% | 250 | 50 | 4 | 5 | 12.5 | 8 | 70% | 61% | 50% |
| Example 3-5 | Ethyl propionate + propyl propionate | 25% + 15% | 100 | 100 | 4 | 1 | 25 | 40 | 75% | 65% | 55% |
| Example 3-6 | Ethyl propionate + propyl propionate | 25% + 15% | 100 | 50 | 4 | 2 | 12.5 | 20 | 76% | 65% | 55% |
| Example 3-7 | Ethyl propionate + propyl propionate | 25% + 15% | 100 | 30 | 4 | 3.3 | 7.5 | 12 | 75% | 65% | 55% |
| Example 3-8 | Ethyl propionate + propyl propionate | 25% + 15% | 100 | 25 | 4 | 4 | 6.3 | 10 | 70% | 60% | 50% |
| Example 3-9 | Ethyl propionate + propyl propionate | 25% + 15% | 100 | 25 | 2 | 4 | 12.5 | 10 | 72% | 62% | 51% |
| Example 3-10 | Ethyl propionate + propyl propionate | 25% + 15% | 100 | 50 | 8 | 2 | 6.3 | 20 | 75% | 65% | 53% |

| | Carboxylate | | Cell size (mm) | | | Length-to-width ratio (L/W) | Width-to-thickness ratio (W/T) | $w_1 \times 100/(L/W)$ | After 250 cycles at 4.5 V at 25°C at different charging rates | | |
|---|---|---|---|---|---|---|---|---|---|---|---|
| | Type | Percentage $w_1$ | Length L | Width W | Thickness T | | | | 0.7C | 1.5C | 3C |
| Example 3-11 | Ethyl propionate + propyl propionate | 25% + 15% | 100 | 50 | 10 | 2 | 5 | 20 | 75% | 65% | 51% |
| Example 3-12 | Ethyl propionate + propyl propionate | 25% + 15% | 100 | 50 | 12 | 2 | 4.2 | 20 | 73% | 60% | 50% |
| Example 3-13 | Ethyl propionate + propyl propionate | 15% + 5% | 100 | 50 | 4 | 2 | 12.5 | 10 | 75% | 65% | 53% |
| Example 3-14 | Ethyl propionate + propyl propionate | 10% + 5% | 100 | 50 | 4 | 2 | 12.5 | 7.5 | 75% | 60% | 49% |
| Example 3-15 | Ethyl acetate + ethyl propionate + propyl propionate | 10% + 20% + 10% | 100 | 50 | 4 | 2 | 12.5 | 20 | 76% | 66% | 55% |
| Example 3-16 | Ethyl acetate + ethyl propionate + propyl propionate | 5% + 10% + 5% | 100 | 50 | 4 | 2 | 12.5 | 10 | 75% | 65% | 54% |
| Example 3-17 | Ethyl acetate + ethyl propionate + propyl propionate | 5% + 5% + 5% | 100 | 50 | 4 | 2 | 12.5 | 7.5 | 75% | 65% | 50% |

The test results in Table 3 indicate that making the battery size design in combination with the percentage of the carboxylate compound in the electrolyte follow a specified relational expression can improve the charging rate window of the battery and alleviate lithium precipitation, thereby improving the fast charging performance of the electrochemical apparatus.

Examples 3-1 to 3-12 indicate that in a case that the electrolyte remains unchanged, when the length-to-width ratio L/W falls within the range of $1 \leq L/W \leq 4$ or the width-to-thickness ratio W/T satisfies $W/T \geq 5$, the fast charging performance of the electrochemical apparatus can be improved, especially the fast charging performance at a high-rate current of above 1.5C can be significantly improved. This is mainly because when the cell falls within the foregoing ranges, current density and temperature rise during charging of upper and lower edge regions and a wound peripheral electrode plate region are slightly different from those of a main body region, reducing lithium precipitation in the edge regions and the peripheral electrode plate region, thereby improving the fast charging performance.

Examples 3-13 to 3-17 indicate that in a case that the cell size remains unchanged, adjusting the percentage of the carboxylate compound in the electrolyte to satisfy $w_1 \times 100/(L/W) \geq 10$ can improve the fast charging performance of the electrochemical apparatus, and especially significantly improve the fast charging performance at a high-rate current of above 1.5C. Although the carboxylate compound added can improve the fast charging performance of the electrochemical apparatus, the high-temperature ITC performance is adversely affected. In addition, based on limitations on a size of the electrochemical apparatus used in a notebook computer, adjustment of carboxylate in combination with the cell size design is used as an effective measure to improve both kinetic performance and battery safety.

In this specification, reference to "an embodiment", "some embodiments", "one embodiment", "another example", "an example", "a specific example", or "some examples" means that at least one embodiment or example in this application includes a specific feature, structure, material, or characteristic described in this embodiment or example. Therefore, descriptions in various places throughout this specification, such as "in some embodiments", "in the embodiments", "in an embodiment", "in another example", "in an example", "in a specific example", or "examples" do not necessarily refer to the same embodiment or example in this application. In addition, a specific feature, structure, material, or characteristic herein may be combined in any appropriate manner in one or more embodiments or examples.

Although illustrative embodiments have been demonstrated and described, persons skilled in the art should understand that the foregoing embodiments are not to be construed as limiting this application, and that the embodiments may be changed, replaced, and modified without departing from the spirit, principle, and scope of this application.

## Claims

1. An electrolyte, comprising a carboxylate compound represented by formula (I) and fluoroethylene carbonate (FEC),

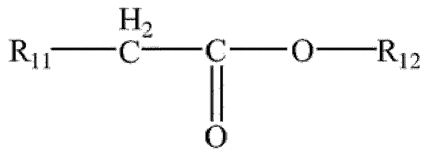

formula (I),

wherein $R_{11}$ includes at least one of hydrogen, a hydroxyl group, a C1-C20 alkyl group, a C1-C20 alkoxy group, a C2-C20 linear alkenyl group, a C6-C30 aryl group, or a C6-C30 aryloxy group, and $R_{12}$ includes at least one of a C1-C20 alkyl group, a C2-C20 linear alkenyl group, or a C6-C30 aryl group; and

based on a total weight of the electrolyte, percentages of the carboxylate compound represented by formula (I) and FEC are $w_1$ and $w_2$ respectively, wherein $5\% \leq w_1 \leq 60\%$, $2\% \leq w_2 \leq 12\%$, and $2 \leq w_1/w_2 \leq 20$.

2. The electrolyte according to claim 1, wherein $4 \leq w_1/w_2 \leq 10$.

3. The electrolyte according to claim 1, wherein the carboxylate compound represented by formula (I) comprises at least one of the following: methyl acetate, ethyl acetate, propyl acetate, methyl propionate, ethyl propionate, propyl propionate, isopropyl propionate, butyl propionate, isobutyl propionate, n-pentyl propionate, isoamyl propionate, ethyl butyrate, propyl butyrate, propyl isobutyrate, amyl butyrate, amyl isobutyrate, butyl butyrate, isobutyl isobutyrate, or pentyl valerate.

4. The electrolyte according to claim 1, wherein the carboxylate compound represented by formula (I) comprises propyl propionate and ethyl acetate.

5. The electrolyte according to claim 1, wherein the electrolyte further comprises a nitrile compound; and based on a total weight of the electrolyte, a percentage of the nitrile compound is $w_3$, wherein $0.1\% \leq w_3 \leq 12\%$.

6. The electrolyte according to claim 5, wherein the nitrile compound comprises at least one of compounds represented by formula (II) to formula (V):

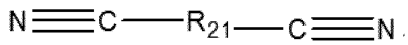

formula (II),

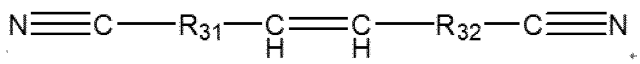

formula (III),

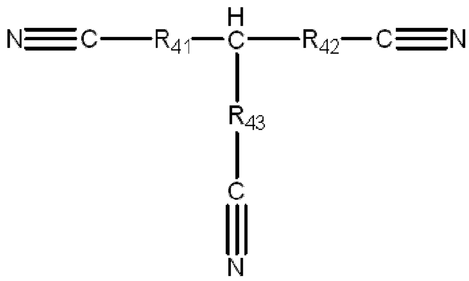

formula (IV), or

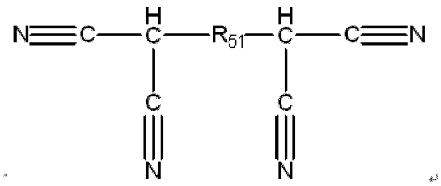

formula (V),

wherein $R_{21}$ comprises at least one of a substituted or unsubstituted C1-C12 alkylidene group or a substituted or unsubstituted C1-C12 alkyleneoxy group;
$R_{31}$ and $R_{32}$ each independently comprise hydrogen and a substituted or unsubstituted C1-C12 alkylidene group;
$R_{41}$, $R_{42}$, and $R_{43}$ each independently comprise hydrogen, a substituted or unsubstituted C1-C12 alkylidene group, or a substituted or unsubstituted C1-C12 alkyleneoxy group; and
$R_{51}$ comprises a substituted or unsubstituted C1-C12 alkylidene group, a substituted or unsubstituted C2-C12 alkenylene group, a substituted or unsubstituted C6-C26 arylidene group, or a substituted or unsubstituted C2-C12 heterocyclylene group, wherein a heteroatom is at least one of N, S, or O; wherein
during substitution, a substituent group is halogen.

**7.** The electrolyte according to claim 5, wherein a total molar mass of cyano groups (-CN) in the nitrile compound is x, a total molar mass of the nitrile compound is y, and cyano group enrichment x/y satisfies $2.16 \leq x/y \leq 2.71$.

**8.** The electrolyte according to claim 7, wherein the cyano group enrichment x/y, the percentage $w_1$ of the carboxylate compound represented by formula (I), and the percentage $w_2$ of FEC satisfy $2w_1^2 - 0.01w_1 + 2.3 > x/y > 27w_2^2 - 1.2w_2 + 2.1$.

**9.** The electrolyte according to claim 1, wherein the electrolyte comprises a lithium salt, and the lithium salt comprises at least one of the following: $LiPF_6$, $LiBF_4$, $LiAsF_6$, $LiClO_4$, $LiB(C_6H_5)_4$, $LiCH_3SO_3$, $LiCF_3SO_3$, $LiN(SO_2CF_3)_2$, $LiC(SO_2CF_3)_3$, $LiSiF_6$, LiBOB, or LiDFOB.

**10.** An electrochemical apparatus, comprising the electrolyte according to any one of claims 1 to 9.

**11.** The electrochemical apparatus according to claim 10, wherein the electrochemical apparatus further comprises a positive electrode, a negative electrode, and a separator sandwiched between the positive electrode and the negative electrode, wherein the positive electrode, the negative electrode, and the separator are wound to form a cell, and a length L and a width W of the cell satisfy 20 mm $\leq$ L $\leq$ 300 mm, 20 mm $\leq$ W $\leq$ 100 mm, and $1 \leq L/W \leq 4$.

**12.** The electrochemical apparatus according to claim 11, wherein the electrochemical apparatus satisfies at least one of the following characteristics: (a) $1 \leq L/W \leq 3$; (b) $2 \leq L/W \leq 3$; and (c) $2 \leq L/W \leq 4$.

**13.** The electrochemical apparatus according to claim 10, wherein the electrochemical apparatus further comprises a positive electrode, a negative electrode, and a separator sandwiched between the positive electrode and the negative electrode, wherein the positive electrode, the negative electrode, and the separator are wound to form a cell, and a thickness T and a width W of the cell satisfy 2 mm $\leq$ T $\leq$ 12 mm, and $W/T \geq 5$.

**14.** The electrochemical apparatus according to claim 13, wherein the electrochemical apparatus satisfies at least one of the following characteristics: (d) $5 \leq W/T \leq 25$; (e) $5 \leq W/T \leq 20$; (f) $5 \leq W/T \leq 15$; and (g) $10 \leq W/T \leq 25$.

**15.** The electrochemical apparatus according to claim 10, wherein the electrochemical apparatus further comprises a positive electrode, a negative electrode, and a separator sandwiched between the positive electrode and the negative electrode, wherein the positive electrode, the negative electrode, and the separator are wound to form a cell, and a length L and a width W of the cell and the percentage $w_1$ of the carboxylate compound represented by formula (I) satisfy $w_1 \times 100/(L/W) \geq 10$.

**16.** The electrochemical apparatus according to claim 15, wherein the electrochemical apparatus satisfies at least one of the following characteristics: (h) $10 \leq w_1 \times 100/(L/W) \leq 40$; (i) $20 \leq w_1 \times 100/(L/W) \leq 30$; (j) $20 \leq w_1 \times 100/(L/W) \leq 40$; and (k) $15 \leq w_1 \times 100/(L/W) \leq 30$.

**17.** An electronic apparatus, comprising the electrochemical apparatus according to any one of claims 10 to 16.

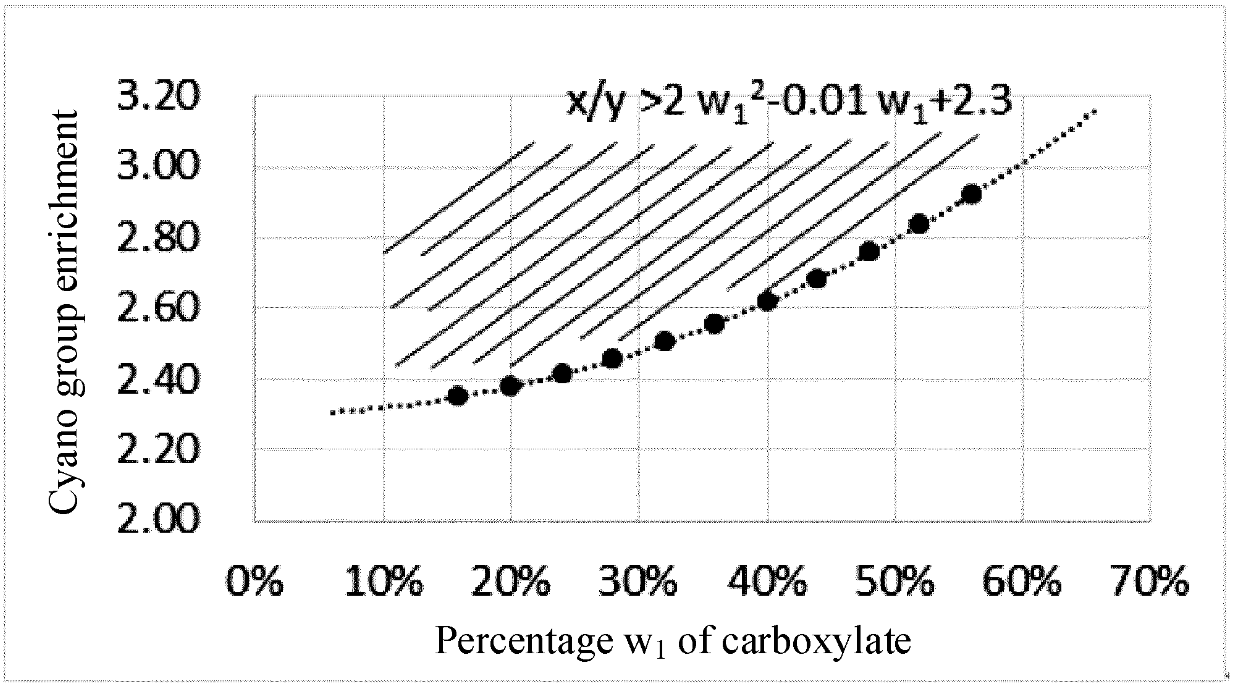
x/y >2 w1²-0.01 w1+2.3
Cyano group enrichment
3.20
3.00
2.80
2.60
2.40
2.20
2.00
0%
10%
20%
30%
40%
50%
60%
70%
Percentage w1 of carboxylate

FIG. 1

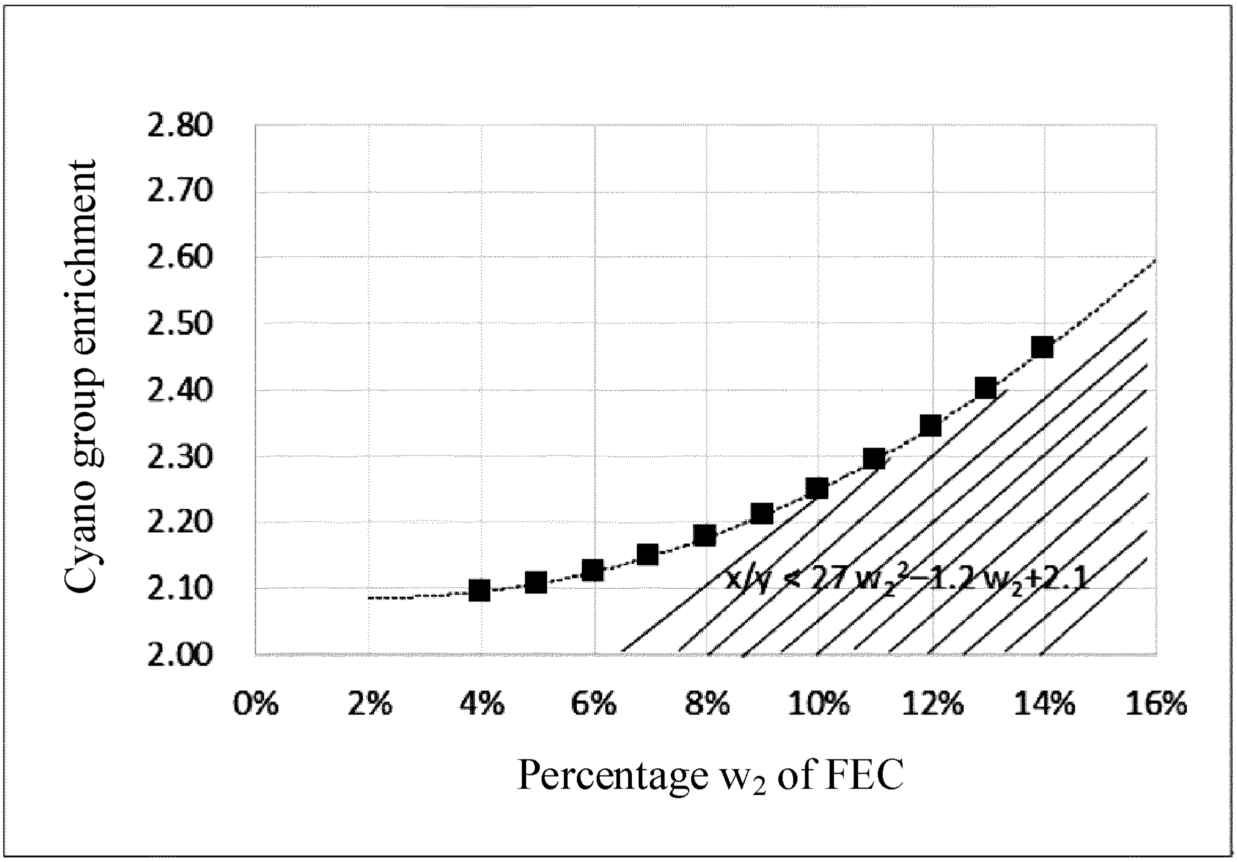
Cyano group enrichment
2.80
2.70
2.60
2.50
2.40
2.30
2.20
2.10
2.00
x/y < 27 w2²−1.2 w2+2.1
0%
2%
4%
6%
8%
10%
12%
14%
16%
Percentage w2 of FEC

FIG. 2

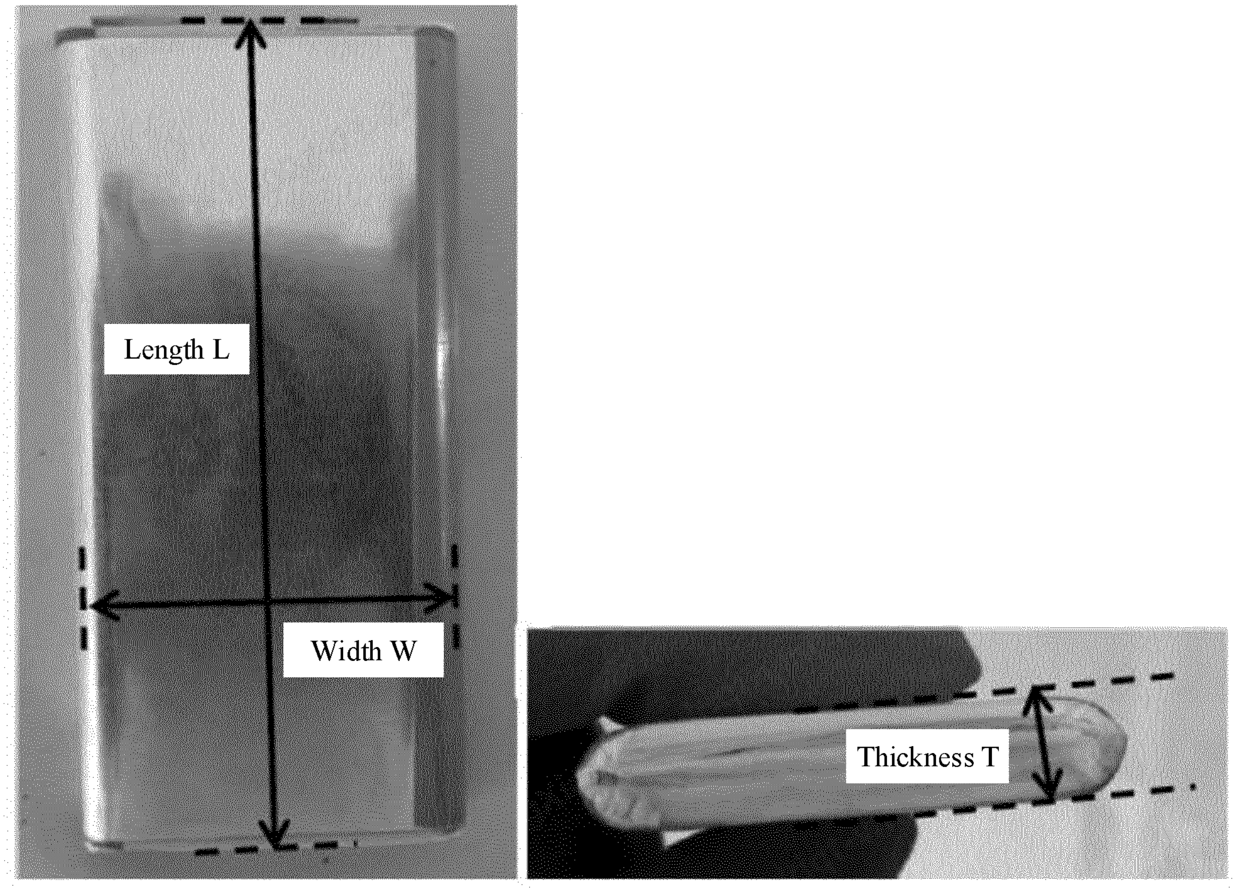


FIG. 3

## INTERNATIONAL SEARCH REPORT

International application No.
**PCT/CN2022/094915**

**A. CLASSIFICATION OF SUBJECT MATTER**

H01M 10/0525(2010.01)i; H01M 10/056(2010.01)i; H01M 10/0567(2010.01)i

According to International Patent Classification (IPC) or to both national classification and IPC

**B. FIELDS SEARCHED**

Minimum documentation searched (classification system followed by classification symbols)

H01M 10/-

Documentation searched other than minimum documentation to the extent that such documents are included in the fields searched

Electronic data base consulted during the international search (name of data base and, where practicable, search terms used)

CNTXT, CNKI, ENTXT, ENTXTC, DWPI, SIPOABS: 电解液, 添加剂, 羧酸酯, 氟代碳酸乙烯酯, 腈, electrolyte, additive, carboxylic ester, fluoroethylene carbonate, nitrile

**C. DOCUMENTS CONSIDERED TO BE RELEVANT**

| Category* | Citation of document, with indication, where appropriate, of the relevant passages | Relevant to claim No. |
|---|---|---|
| X | CN 112349963 A (SHANSHAN ADVANCED MATERIALS (QUZHOU) CO., LTD.) 09 February 2021 (2021-02-09)<br>description, paragraphs 2-31 | 1-17 |
| X | CN 108242556 A (NINGDE CONTEMPORARY AMPEREX TECHNOLOGY CO., LTD.) 03 July 2018 (2018-07-03)<br>description, paragraphs 10-41 | 1-17 |
| X | CN 109193028 A (SHANSHAN ADVANCED MATERIALS (QUZHOU) CO., LTD.) 11 January 2019 (2019-01-11)<br>description, paragraphs 8-30 and 38-100 | 1-17 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 January 2023** | **18 January 2023** |

Name and mailing address of the ISA/CN

**China National Intellectual Property Administration (ISA/CN)**
**No. 6, Xitucheng Road, Jimenqiao, Haidian District, Beijing 100088, China**

Facsimile No. **(86-10)62019451**

Authorized officer

Telephone No.

Form PCT/ISA/210 (second sheet) (January 2015)

**INTERNATIONAL SEARCH REPORT**
**Information on patent family members**

International application No.
**PCT/CN2022/094915**

| Patent document cited in search report | | | Publication date (day/month/year) | Patent family member(s) | | | Publication date (day/month/year) |
|---|---|---|---|---|---|---|---|
| CN | 112349963 | A | 09 February 2021 | CN | 112349963 | B | 22 February 2022 |
| CN | 108242556 | A | 03 July 2018 | WO | 2018120794 | A1 | 05 July 2018 |
| | | | | CN | 108242556 | B | 17 January 2020 |
| CN | 109193028 | A | 11 January 2019 | CN | 109193028 | B | 18 September 2020 |

Form PCT/ISA/210 (patent family annex) (January 2015)